# EUROPEAN PATENT APPLICATION

(11) **EP 1 728 514 A1**
(43) Date of publication of application: **06.12.2006**
(21) Application number: 05104854.4
(22) Date of filing: 03.06.2005
(51) Int. Cl.: A61K 31/7105, A61K 31/711, A61K 31/7115, A61P 17/02, A61P 7/00, A61P 19/10, C12N 15/11

(54) **Uses of oligonucleotides stimulatory of the mesenchymal stem cell proliferation**

(71) Applicant: IMMUNOTECH S.A., Buenos Aires (AR)
(72) Inventor: Lopez, Ricardo Agustin, Buenos Aires (AR)
(74) Representative: Pistolesi, Roberto

(57) **Abstract**

Oligonucleotides having the ability to greatly stimulate the proliferation of pluripotent mesenchymal stem cells "in vitro" and "in vivo" of animals, including humans, are disclosed. These oligonucleotides can be used in a wide range of clinical procedures such as regeneration of mesenchymal tissues which have been damaged through acute injury, abnormal genetic expression or acquired disease by inoculation of the ODNs of this invention.

## Description

This invention relates to the use of oligonucleotides for the manufacture of pharmaceutical compositions which induce "in vitro" and "in vivo" proliferation of MSCs. More in details it refers to oligonucleotides having about 14 to 100 nucleotides that have the ability to greatly stimulate the proliferation of MCSs in animals, including human.

These oligonucleotides have the ability to greatly stimulate the proliferation of pluripotent mesenchymal stem cell "in vitro" and "in vivo" of animals, including humans are disclosed. They can be used in a wide range of clinical procedures such as (1) regeneration of mesenchymal tissues which have been damaged through acute injury, abnormal genetic expression or acquired disease by inoculation of the ODNs of this invention; (2) treatment of a host with damaged mesenchymal tissue by removal of small aliquots of bone marrow, isolation of their mesenchymal stem cells and treatment of the damaged tissue with MSCs culture-expanded by incubation with one or more of the ODNs of this invention combined with a biocompatible carrier suitable for delivering the MSCs to the damaged body site(s); (3) production "in vitro" of various mesenchymal tissues by directed differentiation of the MSCs culture- expanded by incubation with one or more of the ODNs of this invention, to replace and restore tissue damage or defects with say "in vitro" obtained mesenchymal tissues combined with a biocompatible carrier suitable for delivering the "in vitro" produced tissues to the damaged body site(s); and (4) treatment of a host with abnormal genetic expression with MSCs culture- expanded by incubation with one or more of the ODNs of this invention and transformed by genetic engineering procedures to express a protein able to replace the genetic deffect.

Methods for generation of mesenchymal tissues in an area of an animal where say tissues are deficient are provided. They consist in the local or systemic administration of a composition comprising one or more of the oligonucleotides to the animal, in a pharmaceutically acceptable carrier. The composition is administered in an amount effective to induce repair at the damaged site.

Methods for extensive proliferation of MSCs of an animal "in vitro" are also provided. They consist in incubation of small aliquot of bone marrow extracted from the animal, "in vitro" incubation of the cells present in say aliquot of bone marrow in a culture media containing an effective concentration of one or more of the ODNs of this invention for a time period sufficient to obtain the amount of cells necessary for a successful medical treatment.

### BACKGROUND OF THE INVENTION

Mesenchymal stem cells (MSC) also known as marrow stromal cells or mesenchymal progenitor cells, are blast cells found, among other body sites, in bone marrow, blood, dermis and periosteum that are capable of differentiating "in vitro" into bone, cartilage, fat, tendon, muscle, hepatic, renal, cardiac, and neural cells depending upon influences from various bioactive factors (Alhadlaq, A and Mao, J.J. Stem Cells Dev. 13:436, 2004). Also, cultured MSC have the ability to differentiate into different cell specific lineages when placed "in vivo" at the site of a given damaged tissue. Therefore, MSC are a valuable material for medical applications such as systemic transplantation for systemic diseases, local implantation for local tissue defects, gene therapy protocols or generation of transplantable tissues and organs in tissue engineering protocols. In order to isolate MSC, it is necessary to separate them from other cells in the bone marrow or other MSC source. Bone marrow cells may be obtained from iliac crest, femora, tibiae, spine, rib or other medullar spaces. Other sources of MSC include embryonic yolk sac, placenta, umbilical cord, fetal and adolescent skin, blood and other mesenchymal stem cell tissues. A number of processes have been developed for isolation, purification and replication of MSC in cultures (Alhadlaq, A and Mao, J.J. Stem Cells Dev. 13:436, 2004). However, limited proliferative potential of MSC in addition to a very low number in the bone marrow or other sources have so far limited their clinical applications. Immortalization of MSC using viral transforming proteins has been described. However, transformed cells are unlikely to be accepted for standard medical treatments own to its possible tumor inducing potential.

### DESCRIPTION OF THE INVENTION

We now disclose that oligonucleotides having about 14 to 100 nucleotides, preferably from 14 to 40, even more preferably from 14 to 26, are compounds with potent activity to stimulate the proliferation of pluripotent mesenchymal stem cell of animals, including humans, "in vitro" and "in vivo".

According to one embodiment of the invention, a preferred oligonucleotide has at least one subsequence with the following composition: PyNTTTTNT, wherein Py is C or T and wherein N is any deoxyribonucleotide.

Suitable oligonucleotides may be selected from the group consisting of:
TCATCATTTTGTCATTTTGTCATT (SEQ ID N°2);
TCCTCCTTTTGTCCTTTTGTCCTT (SEQ ID N°3);
TCTTCTTTTTGTCTTTTTGTCTTT (SEQ ID N°4);
TTGTTGTTTTGTTGTTTTGTTGTT (SEQ ID N°7);
TCATTTTTTTGTTTTTTTGTCATT (SEQ ID N°10);
TCATTGTTTTGTTGTTTTGTCATT (SEQ ID N°11);
TCATTCTTTTGTTCTTTTGTCATT (SEQ ID N°12);
TCATTATTTTGTTATTTTGTCATT (SEQ ID N°15);
TCATCCTTTTGTCCTTTTGTCATT (SEQ ID N°17);
TCATCTTTTTGTCTTTTTGTCATT (SEQ ID N°18);
CATTTTGTTTTTTTTTTTTTTTTT (SEQ ID N° 20);
TTCATTTTGTTTTTTTTTTTTTTT (SEQ ID N°21);
TTTTCATTTTGTTTTTTTTTTTTT (SEQ ID N°22);
TTTTTTCATTTTGTTTTTTTTTTT (SEQ ID N°23);
TTTTTTTTCATTTTGTTTTTTTTT (SEQ ID N°24);
TTTTTTTTTTCATTTTGTTTTTTT (SEQ ID N°25);
TTTTTTTTTTTTCATTTTGTTTTT (SEQ ID N°26);
TTTTTTTTTTTTTTCATTTTGTTT (SEQ ID N°27);
TTTTTTTTTTTTTTTTCATTTTGT (SEQ ID N° 28);
TTTTCATTTTGTCATTTTGTTTTT (SEQ ID N°29);
TCATCAATTTGTCAATTTGTCATT (SEQ ID N°30);
ACATCATTTTGTCATTTTGTCATT (SEQ ID N°46);
CCATCATTTTGTCATTTTGTCATT (SEQ ID N°47);
GCATCATTTTGTCATTTTGTCATT (SEQ ID N°48);
TAATCATTTTGTCATTTTGTCATT (SEQ ID N°49);
TTATCATTTTGTCATTTTGTCATT (SEQ ID N°50);
TGATCATTTTGTCATTTTGTCATT (SEQ ID N°51);
TCCTCATTTTGTCATTTTGTCATT (SEQ ID N°52);
TCTTCATTTTGTCATTTTGTCATT (SEQ ID N°53);
TCAACATTTTGTCATTTTGTCATT (SEQ ID N°55);
TCACCATTTTGTCATTTTGTCATT (SEQ ID N°56);
TCAGCATTTTGTCATTTTGTCATT (SEQ ID N°57);
TCATCATTTTGTCATTTTGTMTT (SEQ ID N°58);
TCATCATTTTGTCATTTTGTTATT (SEQ ID N°59);
TCATCATTTTGTCATTTTGTGATT (SEQ ID N°60);
TCATCATTTTGTCATTTTGTCCTT (SEQ ID N°61);
TCATCATTTTGTCATTTTGTCTTT (SEQ ID N°62);
TCATCATTTTGTCATTTTGTCAAT (SEQ ID N°64);
TCATCATTTTGTCATTTTGTCACT (SEQ ID N°65);
TCATCATTTTGTCATTTTGTCAGT (SEQ ID N°66);
TCATCATTTTGTCATTTTGTCATA (SEQ ID N°67);
TCATCATTTTGTCATTTTGTCATC (SEQ ID N°68);
TCATCATTTTGTCATTTTGTCATG (SEQ ID N°69);
TCATCAATTGGTCAATTGGTCATT (SEQ ID N°75);
TCATCAACTGGTCAACTGGTCATT (SEQ ID N°77);
TCATCATTGTGTCATTGTGTCATT (SEQ ID N°84);
TCATCATTTGGTCATTTGGTCATT (SEQ ID N°87);
TGCTGCTTTTGTGCTTTTGTGCTT (SEQ ID N°91);
TCATCATCTTGTCATCTTGTCATT (SEQ ID N°95);
TCATCATGTTGTCATGTTGTCATT (SEQ ID N°96);
GGGGGTCTTTTTTTCTTTTTTTTT (SEQ ID N°107);
TTTTTTCTTTTTTTCTTTTTTGGG (SEQ ID N°108);
AAAAATCTTTTTTTCTTTTTTTTT (SEQ ID N°109);
TGCTGCTTTTATGCTTTTATGCTT (SEQ ID N°110);
TCATCATTCTGTCATTCTGTCATT (SEQ ID N°111);
TTTTTTCTTTTTTTCTTTTTTTTT (SEQ ID N°112);
TGCTGCTTTTCTGCTTTTCTGCTT (SEQ ID N°113);
TTTTTTCTTTTCTTTTTTTTTTTT (SEQ ID N°114);
TTTTTCCTTTTTTCCTTTTTTTTT (SEQ ID N°115);
CCCCCTCTTTTTTTCTTTTTTTTT (SEQ ID N°116);
TTTTTTCTTTTTCTTTTTTTTTTT (SEQ ID N°117);
TTTTTTCTTTTTTTCTTTTTTCCC (SEQ ID N°118);
TTTTTTCTTTTTCTCTTTTTCTCT (SEQ ID N°119);
TTTTTGCTTTTTTGCTTTTTTTTT (SEQ ID N°120);
TTTTTACTTTTTTACTTTTTTTTT (SEQ ID N°121);
TCATAATTTTGTAATTTTGTCATT (SEQ ID N°122);
TTTTTTCTTTTTTTCTTTTTTAAA (SEQ ID N°123);
TTTTTTCTTTTTTCTTTTTTTTTT (SEQ ID N°124);
TTTTTTTTTTTTCATTTTGTGGGG (SEQ ID N°125);
TTTTTTTTTTTTCATTTTGTTTTG (SEQ ID N°126);
GGGTTTTTTTTTCATTTTGTTTTT (SEQ ID N°127);
GTTTTTTTTTTTCATTTTGTTTTG (SEQ ID N°128);
TTTTTTTTTTTTCATTTTGTTTGG (SEQ ID N°129);
TTTTTTTTTTTTCATTTTGTTTTA (SEQ ID N°130).
TTTTTTTTTTTTCATTTTGTTTGA (SEQ ID N°131);
TTTTTTTTTTTTCATTTTGTTTAG (SEQ ID N°132);
TTTTTTTTTTTTCATTTTGTTTAA (SEQ ID N°133).

It is an object of the present invention to provide one or more of the oligonucleotides of this invention to an animal with a tissue deficiency to produce normal new normal tissue where it is needed. Another object of the present invention is to provide a method to enhance the replication of pluripotent mesenchymal stem cell "in vitro" so that an adequate number of these cells can be obtained for successful medical treatments.

These objects will become apparent to those skilled in the art.

The first of the above objects is achieved by providing a method for tissue regeneration at a site of an animal where the tissue is deficient and which consists in the administration of an effective amount of a composition comprising one or more of the oligonucleotides of this invention to the animal, locally at the site or systemically as needed in each case, in a pharmaceutically acceptable carrier, the composition being administered in an amount effective to induce tissue regeneration at the site.

This aspect of the invention enables the generation of normal tissue in general or locally as required. Pre-clinical results using as example some of the osteogenic ODNs of this invention described below show tissue formation in tissue defects in rats.

The second of the above objects is achieved by providing a method to enhance the replication of pluripotent mesenchymal stem cell "in vitro" which consists in obtention of bone marrow cells from a suitable source like iliac crest, femora, tibiae, spine, rib or other medullar spaces, culturing these cells "in vitro" using a culture medium to the art supplemented with one or more of the oligonucleotides of this invention for a time period sufficient to obtain the amount of cells necessary for a successful medical treatment.

In particular, the oligonucleotides according to the present invention can be used for the manufacture of a medicament for the treatment of the tissue damage own to a heart stroke, for the treatment of skeletal deficiency or alteration, for the treatment of the tissue damage own to a cartilage deficiency or alteration, for the treatment of the tissue damage own to a nervous system deficiency or alteration, for the treatment of the tissue damage own to a adipose tissue deficiency or alteration, for the treatment of the tissue damage own to a liver deficiency or alteration, for the treatment of the tissue damage own to a muscle deficiency or alteration, for the treatment of the tissue damage own to a skin deficiency or alteration, for the treatment of the tissue damage own to a bum accident, for the treatment of the tissue damage own to a pancreas deficiency or alteration, for the treatment of the tissue damage own to a mucosal deficiency or alteration, for the treatment of the tissue damage own to a blood vessel deficiency or alteration, for the treatment of the tissue damage own to a blood deficiency or alteration and/or for improvement of bone marrow engraftment.

The oligonucleotides for use in the present invention may have any kind of modification of the natural (phosphodiester) phosphate backbone, as for instance a phosphate backbone modification on the 5' inter-nucleotide linkages or a phosphate backbone modification on the 3' inter-nucleotide linkages, or they may have at least one of the internucleotide linkages as a phosphorotioate linkage.

The medicament containing at least one of the above discussed oliconucleotides is preferably administered to a human, the oligonucleotide being preferably present in amounts of 1 µg to 100mg per dose. The medicament may be administered by intradermic, intramuscular or intravenous injection or by oral, intranasal, anal, vaginal, or trans-dermal route; the medicament may be selected from the group consisting of liquid, gel and lyophilized formulations. The oligonucleotide may be included in a pharmaceutically acceptable carrier and/or may be encapsulated in a slow release delivery vehicle.

The oligonucleotide may also be administered to a subject combined with a device aimed to aid in the treatment of a tissue or organ deficiency or alteration.

According to an embodiment, the mesenchymal stem cells are obtained from bone marrow or from periosteum. According to a further embodiment, the mesenchymal stem cells differentiate into cells of more than one tissue cell type, said tissue being preferably selected from bone, cartilage, adipose, tendon, ligament, dermis, epidermis, neuronal, cardiac, renal, glandular and hepatic tissue.

### BRIEF DESCRIPTION OF THE DRAWINGS

Fig. 1a shows the replication of MSCs extracted from the femoral bone marrow of rats stimulated or not in the presence of ODN 504 (SEQ ID N°2).
Fig 1b shows the MSC morphology of cells in controls and ODN IMT504 treated cultures.
Fig 2a shows adipocytes, fibroblasts and macrophages in LTBM culture supplemented with ODN IMT504.
Fig 2b shows calcium deposits in osteoblasts obtained from rat bone marrow cells in osteogenic medium supplemented with ODN IMT504.
Fig 3 shows the MSCs replication capacity in cultures supplemented with CpG ODNs prototypes ODN 2006 and ODN 2216 as compared with the MSCs replication capacity in a culture supplemented with ODN 504.
Fig 4 shows MSCs colony forming units obtained by incubation of bone marrow cells of rats inoculated or not with ODN 504.
Fig 5a shows the radiographic evolution of a defect experimentally provoked in the tibial bone of a rat injected with the ODN IMT504 as compared with a rat injected with placebo after 4 weeks of treatment.
Fig 5b shows the histological analysis of a defect experimentally provoked in the tibial bone of a rat injected with the ODN IMT504 as compared with a rat injected with placebo after 4 weeks of treatment.

### DESCRIPTION OF THE PREFERRED EMBODIMENTS

Repairing a tissue means promoting the formation of morphologically normal, mature tissue only at a site where there is a tissue deficiency that needs to be replaced.

By "animal" is meant any animal having a vertebrate structure, preferably a mammal, and most preferably a human.

A "subject" refers to an animal of the order primate, including humans.

As used herein, the term "oligonucleotide" or "oligo" shall mean multiple nucleotides (i.e. molecules comprising a sugar, e.g. ribose or deoxyribose, linked to a phosphate group and to an exchangeable organic base, which is either a substituted pyrimidine (e.g. cytosine (C), thymine (T) or uracil (U)) or a substituted purine (e.g. adenine (A) or guanine (G)). The term "oligonucleotide" as used herein refers to both oligoribonucleotides (ORNs) and oligodeoxyribonucleotides (ODNs). The term "oligonucleotide" shall also include oligonucleosides (i.e. an oligonucleotide minus the phosphate) and any other organic base containing polymer. Oligonucleotides can be obtained from existing nucleic acid sources (e.g. genomic or cDNA), but are preferably synthetic (e.g. produced by oligonucleotide synthesis).

An "oligonucleotide" refers to multiple nucleotides linked by phosphodiester bonds.

An "immunostimulatory oligonucleotide" refers to an oligonucleotide which stimulates (i.e. has a mitogenic effect on, induces, increases or decreases cytokine expression by) a cell of the immune system (i.e. a lymphocyte or a macrophage) in a statistically significant manner.

A "CpG" refers to a cytosine-guanine dinucleotide.

A "CpG oligonucleotide" refers to an oligonucleotide which stimulates a cell of the immune system, and whose immunostimulatory activity critically depends on the presence of at least one CpG in its sequence.

A "non-CpG oligonucleotide" refers to an oligonucleotide that stimulates a cell of the immune system, and whose immunostimulatory activity does not critically depend on the presence of a CpG in its sequence.

### Modes for Carrying Out the Invention

### EXAMPLE 1

### Materials and Methods

The following materials and methods were generally used throughout the examples.

### 1) Oligonucleotides

Oligonucleotides having phosphorothioate internucleotide linkages were purchased, purified by high-pressure liquid chromatography (HPLC), from Operon Technologies (Alameda, California) or Annovis (Aston, Pennsylvania) or Oligos Etc (Bethel, Maine). ODNs were suspended in depyrogenated water, assayed for LPS contamination using the Limulus test and kept at -20 °C until used. Purity was assessed by HPLC and PAGE assays. ODN preparations were used if LPS levels were undetectable.

### 2) Animal Experiments

2a- Bone marrow (BM) extraction: Rat BM-derived MSCs were harvested from 8 to 12 weeks old (about 350 g) male Spragüe Dawley rats. Animals were anesthetized by intraperitoneal ( i. p.) injection of a mixture of ketamine (50 mg/kg) and xilacine (5 mg/kg). After removing epiphyses and gaining access to the marrow cavities, whole BM plugs were flushed out from femoral bones using a 1 ml syringe with α-MEM medium supplemented with 100 IU/ml gentamicin and 25 µg/ml amphotericin.
2b- "In vivo" MSCs replication stimulation by oligonucleotide treatment: Young (8-12 weeks old) adult male Spragüe Dawley rats weighing about 350 g were subcutaneously injected with 300 µl of PBS containing 250 µg of the oligonucleotide IMT504 (Seq. ID N°2) (treated animals) or 300 µl of PBS (control animals) once a day for five days. Two days after this, the animals were sacrificed and the BM-derived MSCs extracted as described above.
2c- "In vivo" osteogenesis stimulation by oligonucleotide treatment: Young (8-12 weeks old) adult male Spragüe Dawley rats weighing about 350 g were used. Animals were anesthetized by intraperitoneal ( i. p.) injection of a mixture of ketamine (50 mg/kg) and xilacine (5 mg/kg). After skin overlying the hind limb was shaved and sterilized. A 1.5 cm longitudinal incision was performed in the tibia forefront zone. To generate a defect in the bone, an osteotomy was made using a low-speed dental drill attached to a round diamond saw under saline irrigation. For the osteotomy, a site free of muscular insertions was selected 15 mm below the ankle. The wound was deep enough to reach the bone marrow. A single dose (as stated in each experiment) of ODN in 3 µl of methylcellulose 1% was introduced into the defect made in the right tibia. As a control, the same volume (3 µl) of vehicle was introduced into the defect made in the left tibia. Fracture callus formation was evaluated radiographically on days 0, 21 and 28. On day 28, animals were euthanized under ether atmosphere, and the tibias removed and photographed. After this, the tibias were fixed in 10% formol solution, decalcified in 10% EDTA solution, and embedded in paraffin. A longitudinal section of each tibia was cut, stained with the Masson's trichromic technique to visualize calcified zones and examined under a light microscope.

### 3) Tissue culture

3a- MSCs colony forming units determination ( Castro-Malaspina H., Gay R.E., Resnick G., Kapoor N., Meyers P., Chiarieri D., McKenzie S., Broxmeyer H.E., Moore M.A. Characterization of human bone marrow fibroblast colony-forming cells (CFU-F) and their progeny Blood. 1980; 56(2): 289-301): 2 × 10⁶ cells extracted from femoral bones as described above were cultured in 25 cm² culture dish containing 10 ml of α-MEM medium supplemented with 100 IU/ml gentamicin, 25 µg/ml amphotericin, 2 mM L-glutamine and 20% fetal calf serum. Incubation was in 5% CO2 at 37°C.After seven days unattached cells were wash out and fresh medium added. ODN treated cultures contained 1µM of the indicated ODN during the first seven days of culture. Incubation proceeded until day 14. After this, attached cells were washed twice with PBS, fixed with methanol and stained using the May-Grungwald-Giemsa technique. Colonies were observed and counted using an optical microscope.
3b- Long Term Bone Marrow (LTBM) culture: This culture technique was used in order to asses the differentiation capacity of the cultured MSCs (Gartner S., Kaplan H.S. Long-term culture of human bone marrow cells. Proc Natl Acad Sci U S A. 1980; 77(8): 4756-4759). 10 × 10⁶ extracted from femoral bones as described above were cultured in 25 cm² culture dish containing 10 ml of α-MEM medium supplemented with 100 IU/ml gentamicin, 25 µg/ml amphotericin, 2 mM L-glutamine, 12,5 % fetal calf serum, 12,5% horse serum, and 10⁻⁸ M hydrocortisone. Incubation was in 5% CO2 at 37°C for 28 days. Unattached cells were eliminated every seven days by centrifugation of the cell supernatant. After this, half of the supernatant was replaced by fresh medium and returned to the culture dish. ODN treated cultures contained 1 µM of the indicated ODN during the first seven days of culture. After this, the stimulus was progressively diluted with the medium replacement. After incubation, attached cells were washed twice with PBS, fixed with methanol and stained using the May-Grunwald-Giemsa technique. Cells were observed using an optical microscope.
3c- Osteogenic differentiation of the MSCs "in vitro" (Bruder S.P, Jaiswal N., Haynesworth S.E. Growth kinetics, self-renewal, and the osteogenic potential of purified human mesenchymal stem cells during extensive subcultivation and following cryopreservation. J Cell Biochem. 1997; vol. 64, p. 278): 2 × 10⁶ cells extracted from femoral bones as described above were cultured in 25 cm² culture dish containing 10 ml of D-MEM medium supplemented with 100 IU/ml gentamicin, 2.5 µg/ml amphotericin, 2 mM L-glutamine and 10% fetal calf serum. Cultures also contained 1 µM of the indicated ODN. After three days culture unattached cells were wash out and fresh medium and ODN were added. After seven days more of culture, cells were trypsinized and seeded in a new culture dish at 3000 cells/cm² containing the differentiating medium. This medium consisted in D-MEM supplemented with 10-8 dexametazone, 0.2 mM ascorbic acid and 10mM β-glicerophosphate. The cell supernatant was replaced every three days with fresh medium. After 21 days attached cells were washed twice with PBS, fixed with methanol and stained using the Alizarin Red technique to visualize calcium deposits.

### EXAMPLE 2

### ODN stimulation of the MSCs replication "in vitro"

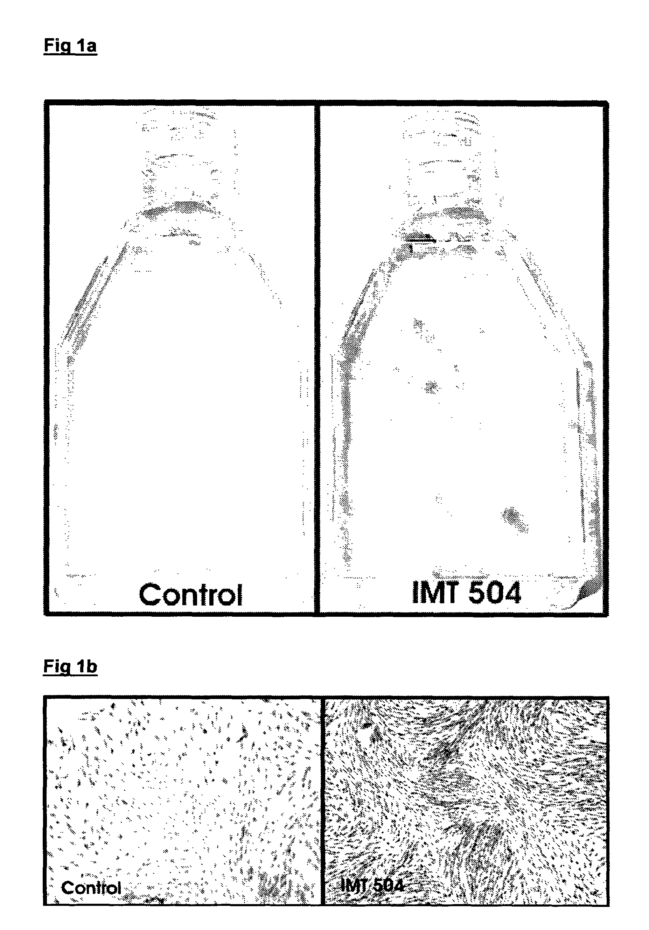

The oligonucleotide used in these experiments was IMT504 (SEQ ID N°2). This oligonucleotide is 24 nucleotides long; its nucleotide sequence is 5'-TCATCATTTTGTCATTTTGTCATT-3', and all the DNA (natural) phosphodiester bonds have been replaced with phosphorothioate bonds to protect it from enzymatic degradation.Figure 1a shows that the replication of MSCs extracted from the femoral bone marrow of rats are greatly stimulated in the presence of ODN 504. Fig 1b shows the typical MSC morphology of cells in controls and treated cultures. Many other oligonucleotides (e.g.: SEQ ID N°3, SEQ ID N°4, SEQ ID N°7 and SEQ ID N°10) were assayed with similar results.

### EXAMPLE 3

### MSCs replicated "in vitro" under ODN stimulation conserve the multipotent differentiation capacity

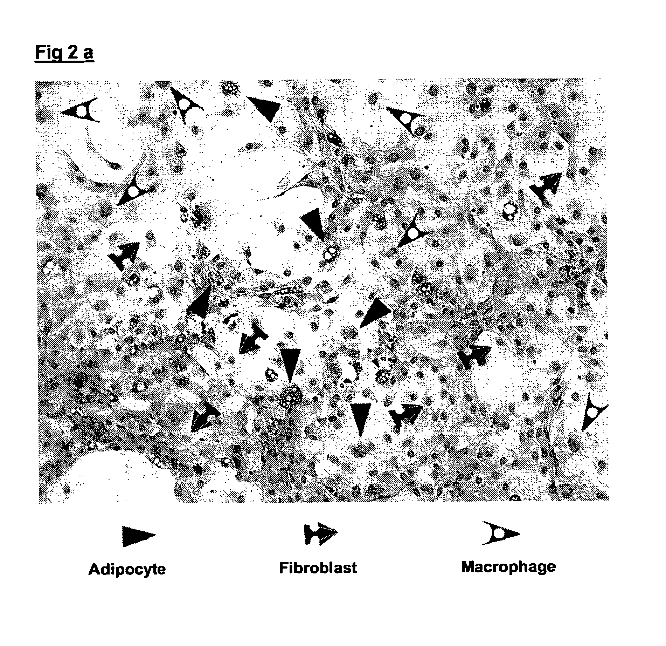

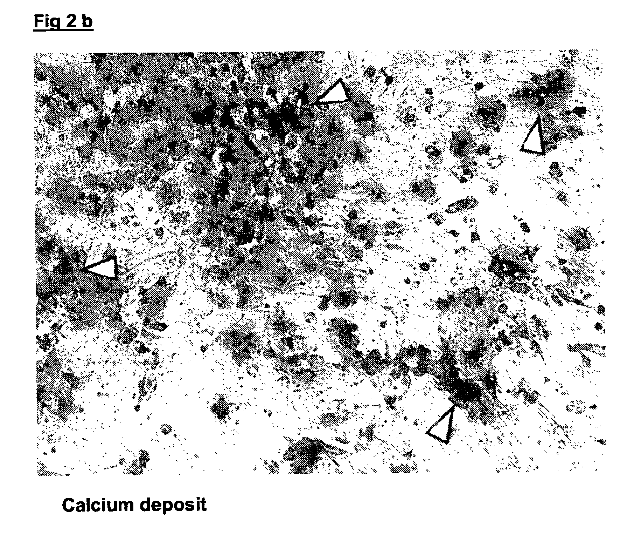

Fig 2 a shows that MSCs replicated "in vitro" under ODN stimulation could differentiate in LTBM culture to adipocytes, fibroblasts and macrophages as expected for normal MSCs (Gartner S., Kaplan H.S. Long-term culture of human bone marrow cells. Proc Natl Acad Sci U S A. 1980; 77(8): 4756-4759). On the other hand, Fig 2 b shows that in osteogenic medium (Bruder S.P, Jaiswal N., Haynesworth S.E. Growth kinetics, self-renewal, and the osteogenic potential of purified human mesenchymal stem cells during extensive subcultivation and following cryopreservation. J Cell Biochem. 1997; vol. 64, p. 278) MSCs replicated "in vitro" under ODN stimulation could differentiate into osteoblasts that form calcium deposits.

### EXAMPLE 4

### CpG ODNs are poor stimulators of the MSCs replication "in vitro"

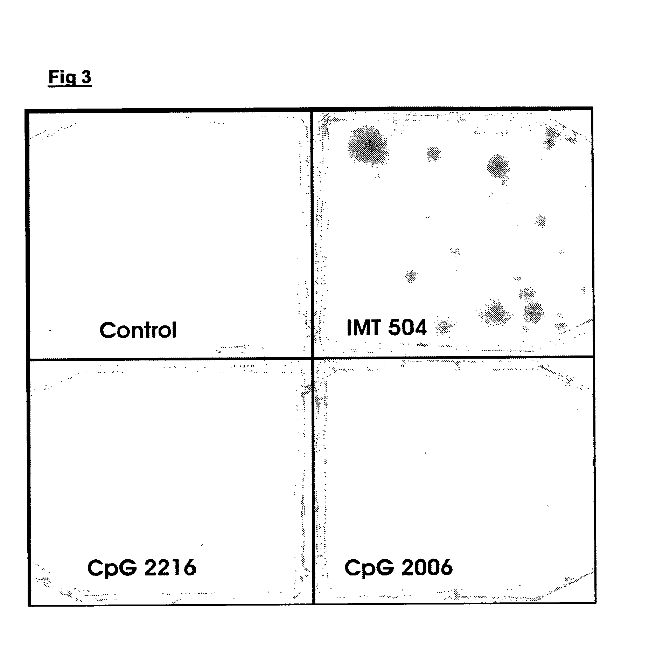

1. Fig 3 shows that CpG ODNs prototypes ODN 2006 and ODN 2216 (Krieg, A.M. GpG motifs in bacterial DNA and their immune effects. 2002. Annu. Rev. Immunol. 20, 709-760) are poor stimulators of the MSCs replication as compared with ODN 504. Many other CpG ODNs were assayed with identical results.

### EXAMPLE 5

### ODN stimulation of the MSCs replication "in vivo"

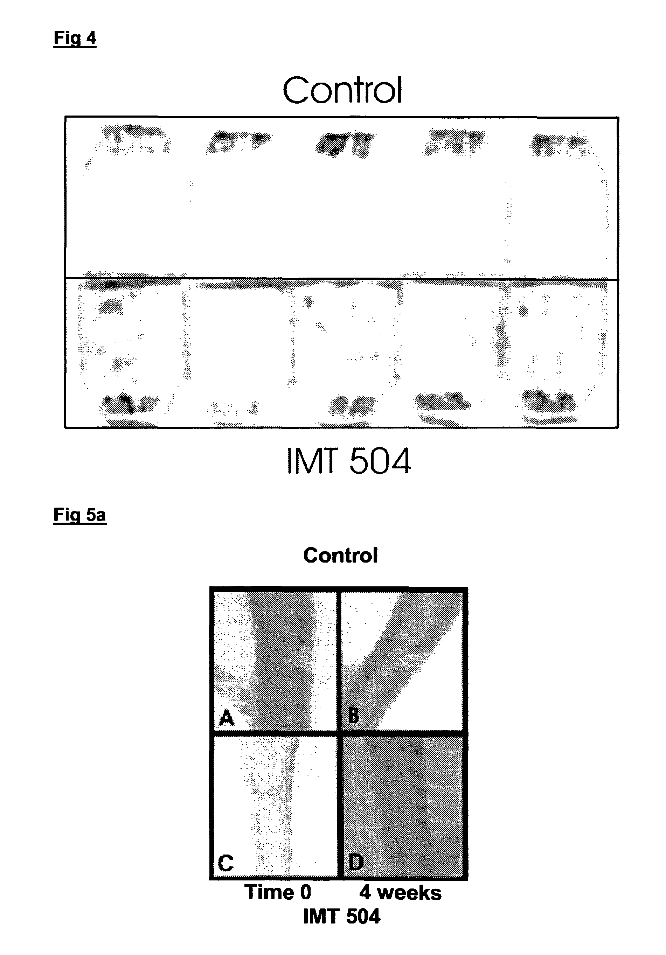

Fig 4 shows that bone marrow of rats inoculated with ODN 504 have a very high level of MSCs colony forming cells as compared with rats inoculated with placebo. This result indicates that the stimulation of the MSCs replication by ODNs also occurs "in vivo".

### EXAMPLE 6

### ODN stimulation of the osteogenesis "in vivo"

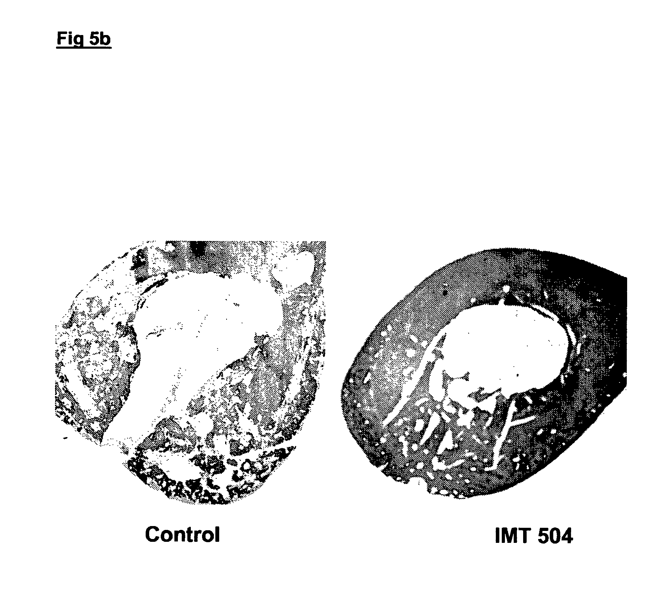

Since stimulation of the replication of MSCs could be induced by the ODN and since MSCs are osteogenic when injected in an animal (Bruder SP, Fink DJ, Caplan AI. Mesenchymal stem cells in bone development, bone repair, and skeletal regeneration therapy. J Cell Biochem. 1994 Nov;56(3):283-94. Review), the osteogenic potential of the MSCs over-replicated "in vivo" by ODN injection was assayed. Fig 5a and 5b shows that an experimental defect provoked in the tibial bone of rats is rapidly repaired in animals treated with the ODN IMT504 as compared with rats injected with placebo.

### RELEVANT REFERENCES

### U.S. Patent Documents

| **General application of MSCs** | |
|---|---|
| United States Patent 6,387,367 | Human mesenchymal stem cells |
| United States Patent 5,827,735 | Pluripotent mesenchymal stem cells and methods of use thereof |
| United States Patent 5,486,359 | Human mesenchymal stem cells |
| US Patent (appl.) 20040258669 | Mesenchymal stem cells and methods of use thereof |
| US Patent (appl.) 20040048375 | Application of pluripotential stem cells for body organ tissue repair and venous capillary expansion |

| **MSCs isolation methods** | |
|---|---|
| US Patent (appl.) 20030211602 | Isolation of mesenchymal stem cells and use thereof |

| **Use of MSCs in bone regeneration** | |
|---|---|
| United States Patent 6,863,900 | Regeneration and augmentation of bone using mesenchymal stem cells. |
| United States Patent 6,541,024 | Regeneration and augmentation of bone using mesenchymal stem cells |
| US Patent (appl.) 20030031695 | Regeneration and augmentation of bone using mesenchymal stem cells |

| **Use of MSCs in regeneration of adipose tissue** | |
|---|---|
| United States Patent 6,709,864 | Adipogenic differentiation of human mesenchymal stem cells |
| United States Patent 6,322,784 | Adipogenic differentiation of human mesenchymal stem cells |
| United States Patent 5,827,740 | Adipogenic differentiation of human mesenchymal stem cells |

| **Use of MSCs in regeneration of liver tissue** | |
|---|---|
| United States Patent 6,458,589 | Hepatocyte lineage cells derived from pluripotent stem cells |

| **Use of MSCs in regeneration of cardiac tissue** | |
|---|---|
| United States Patent 6,387,369 | Cardiac muscle regeneration using mesenchymal stem cells |
| US Patent (appl.) 20040180043 | Cardiac transplantation of stem cells for the treatment of heart failure |
| US Patent (appl.) 20030103951 | Cardiac muscle regeneration using mesenchymal stem cells |

| **Use of MSCs cartilage repair** | |
|---|---|
| United States Patent 6,214,369 | Mesenchymal stem cells for cartilage repair |
| United States Patent 5,908,784 | In vitro chondrogenic induction of human mesenchymal stem cells |
| United States Patent 5,906,934 | Mesenchymal stem cells for cartilage repair |
| US Patent (appl.) 20030026786 | Chondrogenic differentiation of human mesenchymal stem cells |
| US Patent (appl.) 20020005205 | Joint repair using mesenchymal stem cells |

| **Use of MSCs in regeneration of pancreatic tissue** | |
|---|---|
| US Patent (appl.) 20050054102 | Method for differentiating stem cells into insulin-producing cells |

| **Use of MSCs in regeneration of neural tissue** | |
|---|---|
| US Patent (appl.) 20040151701 | Method for differentiating mesenchymal stem cells into neural cells |
| US Patent (appl.) 20040115806 | Method of generating neurons from stem cells and medium for culturing stem cells |

| **Use of MSCs in prevention of the immune response in transplantation** | |
|---|---|
| US Patent (appl.) 20020085996 | Mesenchymal stem cells for prevention and treatment of immune responses in transplantation |

| **Use of MSCs in skin regeneration** | |
|---|---|
| AU000001832601a | Skin Regeneration Using Mesenchymal Stem Cells |

| **Use of MSCs in muscle regeneration** | |
|---|---|
| AU000000706026b | Myogenic Differentiation Of Human Mesenchymal Stem Cells |

SEQUENCE LISTING

<110> IMMUNOTECH S.A.

<120> Oligonucleotides stimulatory of the mesenchymal stem cell
proliferation and uses thereof

<130> 05mg67e

<140> EP05104854.4
<141> 2005-06-03

<160> 133

<170> PatentIn version 3.3

<210> 1
<211> 24
<212> DNA
<213> Artificial

<220>
<223> Synthetic Construct

<400> 1
tgctgctttt gtgcttttgt gctt 24


<210> 2
<211> 24
<212> DNA
<213> Artificial

<220>
<223> Synthetic Construct

<400> 2
tcatcatttt gtcattttgt catt 24


<210> 3
<211> 24
<212> DNA
<213> Artificial

<220>
<223> Synthetic Construct

<400> 3
tcctcctttt gtccttttgt cctt 24


<210> 4
<211> 24
<212> DNA
<213> Artificial

<220>
<223> Synthetic Construct

<400> 4
   tcttcttttt gtctttttgt cttt 24


<210> 5
<211> 24
<212> DNA
<213> Artificial

<220>
<223>  Synthetic Construct

<400>  5
tagtagtttt gtagttttgt agtt                                              24


<210>  6
<211>  24
<212>  DNA
<213>  Artificial

<220>
<223>  Synthetic Construct

<400>  6
tggtggtttt gtggttttgt ggtt                                              24


<210>  7
<211>  24
<212>  DNA
<213>  Artificial

<220>
<223>  Synthetic Construct

<400>  7
ttgttgtttt gttgttttgt tgtt                                              24


<210>  8
<211>  24
<212>  DNA
<213>  Artificial

<220>
<223>  Synthetic Construct

<400>  8
tgctgcaaaa gagcaaaaga gcaa                                              24


<210>  9
<211>  24
<212>  DNA
<213>  Artificial

<220>
<223>  Synthetic Construct

<400>  9
tgctgccccc gcgcccccgc gccc                                              24


<210>  10
<211>  24
<212>  DNA
<213>  Artificial

<220>
<223>  Synthetic Construct

<400>  10
tcattttttt gtttttttgt catt                                              24

<210>  11
<211>  24
<212>  DNA
<213>  Artificial

<220>
<223>  Synthetic Construct

<400>  11
tcattgtttt gttgttttgt catt                                              24


<210>  12
<211>  24
<212>  DNA
<213>  Artificial

<220>
<223>  Synthetic Construct

<400>  12
tcattctttt gttcttttgt catt                                              24


<210>  13
<211>  24
<212>  DNA
<213>  Artificial

<220>
<223>  Synthetic Construct

<400>  13
aaaaaactaa aaaaaactaa aaaa                                              24


<210>  14
<211>  24
<212>  DNA
<213>  Artificial

<220>
<223>  Synthetic Construct

<400>  14
tcataatttt gtaattttgt catt                                              24


<210>  15
<211>  24
<212>  DNA
<213>  Artificial

<220>
<223>  Synthetic Construct

<400>  15
tcattatttt gttattttgt catt                                              24


<210>  16
<211>  24
<212>  DNA
<213>  Artificial

<220>
<223>  Synthetic Construct

<400>  16
tcatgatttt gtgattttgt catt                                              24


<210>  17
<211>  24
<212>  DNA
<213>  Artificial

<220>
<223>  Synthetic Construct

<400>  17
tcatcctttt gtccttttgt catt                                              24


<210>  18
<211>  24
<212>  DNA
<213>  Artificial

<220>
<223>  Synthetic Construct

<400>  18
tcatcttttt gtcttttgt catt                                               24


<210>  19
<211>  24
<212>  DNA
<213>  Artificial

<220>
<223>  Synthetic Construct

<400>  19
tttttttttt tttttttttt tttt                                              24


<210>  20
<211>  24
<212>  DNA
<213>  Artificial

<220>
<223>  Synthetic Construct

<400>  20
cattttgttt tttttttttt tttt                                              24


<210>  21
<211>  24
<212>  DNA
<213>  Artificial

<220>
<223>  Synthetic Construct

<400>  21
ttcattttgt tttttttttt tttt                                              24


<210>  22
<211>  24
<212>  DNA
<213>  Artificial

<220>
<223> Synthetic Construct

<400> 22
ttttcatttt gttttttttt tttt 24

<210> 23
<211> 24
<212> DNA
<213> Artificial

<220>
<223> Synthetic Construct

<400> 23
ttttttcatt ttgttttttt tttt 24

<210> 24
<211> 24
<212> DNA
<213> Artificial

<220>
<223> Synthetic Construct

<400> 24
ttttttttca ttttgttttt tttt 24

<210> 25
<211> 24
<212> DNA
<213> Artificial

<220>
<223> Synthetic Construct

<400> 25
tttttttttt cattttgttt tttt 24

<210> 26
<211> 24
<212> DNA
<213> Artificial

<220>
<223> Synthetic Construct

<400> 26
tttttttttt ttcattttgt tttt 24

<210> 27
<211> 24
<212> DNA
<213> Artificial

<220>
<223> Synthetic Construct

<400> 27
tttttttttt ttttcatttt gttt 24

<210> 28
<211> 24
<212> DNA
<213> Artificial

<220>
<223> Synthetic Construct

<400> 28
tttttttttt ttttttcatt ttgt 24


<210> 29
<211> 24
<212> DNA
<213> Artificial

<220>
<223> Synthetic Construct

<400> 29
ttttcatttt gtcattttgt tttt 24


<210> 30
<211> 24
<212> DNA
<213> Artificial

<220>
<223> Synthetic Construct

<400> 30
tcatcaattt gtcaatttgt catt 24


<210> 31
<211> 24
<212> DNA
<213> Artificial

<220>
<223> Synthetic Construct

<400> 31
tcatcatatt gtcatattgt catt 24


<210> 32
<211> 24
<212> DNA
<213> Artificial

<220>
<223> Synthetic Construct

<400> 32
tcatcattat gtcattatgt catt 24


<210> 33
<211> 24
<212> DNA
<213> Artificial

<220>
<223> Synthetic Construct

<400>  33
tcatcattta gtcatttagt catt                                              24


<210>  34
<211>  24
<212>  DNA
<213>  Artificial

<220>
<223>  Synthetic Construct

<400>  34
tcatcatttt atcattttat catt                                              24


<210>  35
<211>  24
<212>  DNA
<213>  Artificial

<220>
<223>  Synthetic Construct

<400>  35
tcatcatttt ttcatttttt catt                                              24


<210>  36
<211>  24
<212>  DNA
<213>  Artificial

<220>
<223>  Synthetic Construct

<400>  36
tcatcatttt ctcattttct catt                                              24


<210>  37
<211>  24
<212>  DNA
<213>  Artificial

<220>
<223>  Synthetic Construct

<400>  37
tcatcatttt gacattttga catt                                              24


<210>  38
<211>  24
<212>  DNA
<213>  Artificial

<220>
<223>  Synthetic Construct

<400>  38
tcatcattta gacatttaga catt                                              24


<210>  39
<211>  24
<212>  DNA
<213>  Artificial

<220>
<223>  Synthetic Construct

<400>  39
tcatcattat gacattatga catt                                              24


<210>  40
<211>  24
<212>  DNA
<213>  Artificial

<220>
<223>  Synthetic Construct

<400>  40
tcatcatatt gacatattga catt                                              24


<210>  41
<211>  24
<212>  DNA
<213>  Artificial

<220>
<223>  Synthetic Construct

<400>  41
tcatcattaa gacattaaga catt                                              24


<210>  42
<211>  24
<212>  DNA
<213>  Artificial

<220>
<223>  Synthetic Construct

<400>  42
tcatcatata gacatataga catt                                              24


<210>  43
<211>  24
<212>  DNA
<213>  Artificial

<220>
<223>  Synthetic Construct

<400>  43
tcatcataat gacataatga catt                                              24


<210>  44
<211>  24
<212>  DNA
<213>  Artificial

<220>
<223>  Synthetic Construct

<400>  44
tcatcataaa gacataaaga catt                                              24

<210>  45
<211>  24
<212>  DNA
<213>  Artificial

<220>
<223>  Synthetic Construct

<400>  45
tcatcaaaaa gacaaaaaga catt                                              24


<210>  46
<211>  24
<212>  DNA
<213>  Artificial

<220>
<223>  Synthetic Construct

<400>  46
acatcatttt gtcattttgt catt                                              24


<210>  47
<211>  24
<212>  DNA
<213>  Artificial

<220>
<223>  Synthetic Construct

<400>  47
ccatcatttt gtcattttgt catt                                              24


<210>  48
<211>  24
<212>  DNA
<213>  Artificial

<220>
<223>  Synthetic Construct

<400>  48
gcatcatttt gtcattttgt catt                                              24


<210>  49
<211>  24
<212>  DNA
<213>  Artificial

<220>
<223>  Synthetic Construct

<400>  49
taatcatttt gtcattttgt catt                                              24


<210>  50
<211>  24
<212>  DNA
<213>  Artificial

<220>
<223>  Synthetic Construct

<400>  50
ttatcatttt gtcattttgt catt                                              24


<210>  51
<211>  24
<212>  DNA
<213>  Artificial

<220>
<223>  Synthetic Construct

<400>  51
tgatcatttt gtcattttgt catt                                              24


<210>  52
<211>  24
<212>  DNA
<213>  Artificial

<220>
<223>  Synthetic Construct

<400>  52
tcctcatttt gtcattttgt catt                                              24


<210>  53
<211>  24
<212>  DNA
<213>  Artificial

<220>
<223>  Synthetic Construct

<400>  53
tcttcatttt gtcattttgt catt                                              24


<210>  54
<211>  24
<212>  DNA
<213>  Artificial

<220>
<223>  Synthetic Construct

<400>  54
tcgtcatttt gtcattttgt catt                                              24


<210>  55
<211>  24
<212>  DNA
<213>  Artificial

<220>
<223>  Synthetic Construct

<400>  55
tcaacatttt gtcattttgt catt                                              24


<210>  56
<211>  24
<212>  DNA
<213>  Artificial

<220>
<223>  Synthetic Construct

<400>  56
tcaccatttt gtcattttgt catt                                              24


<210>  57
<211>  24
<212>  DNA
<213>  Artificial

<220>
<223>  Synthetic Construct

<400>  57
tcagcatttt gtcattttgt catt                                              24


<210>  58
<211>  24
<212>  DNA
<213>  Artificial

<220>
<223>  Synthetic Construct

<400>  58
tcatcatttt gtcattttgt aatt                                              24


<210>  59
<211>  24
<212>  DNA
<213>  Artificial

<220>
<223>  Synthetic Construct

<400>  59
tcatcatttt gtcattttgt tatt                                              24


<210>  60
<211>  24
<212>  DNA
<213>  Artificial

<220>
<223>  Synthetic Construct

<400>  60
tcatcatttt gtcattttgt gatt                                              24


<210>  61
<211>  24
<212>  DNA
<213>  Artificial

<220>
<223>  Synthetic Construct

<400>  61
tcatcatttt gtcattttgt cctt                                              24

<210> 62
<211> 24
<212> DNA
<213> Artificial

<220>
<223> Synthetic Construct

<400> 62
tcatcatttt gtcattttgt cttt 24


<210> 63
<211> 24
<212> DNA
<213> Artificial

<220>
<223> Synthetic Construct

<400> 63
tcatcatttt gtcattttgt cgtt 24


<210> 64
<211> 24
<212> DNA
<213> Artificial

<220>
<223> Synthetic Construct

<400> 64
tcatcatttt gtcattttgt caat 24


<210> 65
<211> 24
<212> DNA
<213> Artificial

<220>
<223> Synthetic Construct

<400> 65
tcatcatttt gtcattttgt cact 24


<210> 66
<211> 24
<212> DNA
<213> Artificial

<220>
<223> Synthetic Construct

<400> 66
tcatcatttt gtcattttgt cagt 24


<210> 67
<211> 24
<212> DNA
<213> Artificial

<220>
<223> Synthetic Construct

<400>  67
tcatcatttt gtcattttgt cata                                              24


<210>  68
<211>  24
<212>  DNA
<213>  Artificial

<220>
<223>  Synthetic Construct

<400>  68
tcatcatttt gtcattttgt catc                                              24


<210>  69
<211>  24
<212>  DNA
<213>  Artificial

<220>
<223>  Synthetic Construct

<400>  69
tcatcatttt gtcattttgt catg                                              24


<210>  70
<211>  12
<212>  DNA
<213>  Artificial

<220>
<223>  Synthetic Construct

<400>  70
ttttcatttt gt                                                           12


<210>  71
<211>  16
<212>  DNA
<213>  Artificial

<220>
<223>  Synthetic Construct

<400>  71
ttttcatttt gttttt                                                       16


<210>  72
<211>  20
<212>  DNA
<213>  Artificial

<220>
<223>  Synthetic Construct

<400>  72
ttttcatttt gttttttttt                                                   20


<210>  73
<211>  24
<212>  DNA
<213>  Artificial

<220>
<223>  Synthetic Construct

<400>  73
tttttttttt ttcattttgt tttt                                              24


<210>  74
<211>  28
<212>  DNA
<213>  Artificial

<220>
<223>  Synthetic Construct

<400>  74
ttttcatttt gttttttttt tttttttt                                          28


<210>  75
<211>  24
<212>  DNA
<213>  Artificial

<220>
<223>  Synthetic Construct

<400>  75
tcatcaattg gtcaattggt catt                                              24


<210>  76
<211>  24
<212>  DNA
<213>  Artificial

<220>
<223>  Synthetic Construct

<400>  76
tcatcaaatg gtcaaatggt catt                                              24


<210>  77
<211>  24
<212>  DNA
<213>  Artificial

<220>
<223>  Synthetic Construct

<400>  77
tcatcaactg gtcaactggt catt                                              24


<210>  78
<211>  24
<212>  DNA
<213>  Artificial

<220>
<223>  Synthetic Construct

<400>  78
tcatcaagtg gtcaagtggt catt                                              24

<210>  79
<211>  24
<212>  DNA
<213>  Artificial

<220>
<223>  Synthetic Construct

<400>  79
tcatcaatag gtcaataggt catt                                              24


<210>  80
<211>  24
<212>  DNA
<213>  Artificial

<220>
<223>  Synthetic Construct

<400>  80
tcatcaatcg gtcaatcggt catt                                              24


<210>  81
<211>  24
<212>  DNA
<213>  Artificial

<220>
<223>  Synthetic Construct

<400>  81
tcatcaatgg gtcaatgggt catt                                              24


<210>  82
<211>  24
<212>  DNA
<213>  Artificial

<220>
<223>  Synthetic Construct

<400>  82
tcatcattat gtcattatgt catt                                              24


<210>  83
<211>  24
<212>  DNA
<213>  Artificial

<220>
<223>  Synthetic Construct

<400>  83
tcatcattct gtcattctgt catt                                              24


<210>  84
<211>  24
<212>  DNA
<213>  Artificial

<220>
<223>  Synthetic Construct

<400>  84
tcatcattgt gtcattgtgt catt                                              24


<210>  85
<211>  24
<212>  DNA
<213>  Artificial

<220>
<223>  Synthetic Construct

<400>  85
tcatcattta gtcatttagt catt                                              24


<210>  86
<211>  24
<212>  DNA
<213>  Artificial

<220>
<223>  Synthetic Construct

<400>  86
tcatcatttc gtcatttcgt catt                                              24


<210>  87
<211>  24
<212>  DNA
<213>  Artificial

<220>
<223>  Synthetic Construct

<400>  87
tcatcatttg gtcatttggt catt                                              24


<210>  88
<211>  24
<212>  DNA
<213>  Artificial

<220>
<223>  Synthetic Construct

<400>  88
tcatcatttt gacattttga catt                                              24


<210>  89
<211>  24
<212>  DNA
<213>  Artificial

<220>
<223>  Synthetic Construct

<400>  89
tcatcatttt gccattttgc catt                                              24


<210>  90
<211>  24
<212>  DNA
<213>  Artificial

<220>
<223>  Synthetic Construct

<400>  90
tcatcatttt ggcattttgg catt                                              24


<210>  91
<211>  24
<212>  DNA
<213>  Artificial

<220>
<223>  Synthetic Construct

<400>  91
tgctgctttt gtgcttttgt gctt                                              24


<210>  92
<211>  24
<212>  DNA
<213>  Artificial

<220>
<223>  Synthetic Construct

<400>  92
tcatcacttt ggcactttgg catt                                              24


<210>  93
<211>  24
<212>  DNA
<213>  Artificial

<220>
<223>  Synthetic Construct

<400>  93
tcatcagttt ggcagtttgg catt                                              24


<210>  94
<211>  24
<212>  DNA
<213>  Artificial

<220>
<223>  Synthetic Construct

<400>  94
tcatcatatt ggcatattgg catt                                              24


<210>  95
<211>  24
<212>  DNA
<213>  Artificial

<220>
<223>  Synthetic Construct

<400>  95
tcatcatctt ggcatcttgg catt                                              24

<210>  96
<211>  24
<212>  DNA
<213>  Artificial

<220>
<223>  Synthetic Construct

<400>  96
tcatcatgtt ggcatgttgg catt                                              24


<210>  97
<211>  24
<212>  DNA
<213>  Artificial

<220>
<223>  Synthetic Construct

<400>  97
tgctgccccc gcgcccccgc gccc                                              24


<210>  98
<211>  24
<212>  DNA
<213>  Artificial

<220>
<223>  Synthetic Construct

<400>  98
ggggtttttt tttttttttt tttt                                              24


<210>  99
<211>  24
<212>  DNA
<213>  Artificial

<220>
<223>  Synthetic Construct

<400>  99
   ggggtctttt tttctttttt tctt                                              24


<210>  100
<211>  24
<212>  DNA
<213>  Artificial

<220>
<223>  Synthetic Construct

<400>  100
tgctgccccc gcgcccccgc gccc                                              24


<210>  101
<211>  24
<212>  DNA
<213>  Artificial

<220>
<223>  Synthetic Construct

<400>  101
ggggactttt atacttttat actt                                              24


<210>  102
<211>  24
<212>  DNA
<213>  Artificial

<220>
<223>  Synthetic Construct

<400>  102
   ggggtatttt tttatttttt tatt                                               24


<210>  103
<211>  24
<212>  DNA
<213>  Artificial

<220>
<223>  Synthetic Construct

<400>  103
gggggctttt gggcttttgg gcgg                                              24


<210>  104
<211>  24
<212>  DNA
<213>  Artificial

<220>
<223>  Synthetic Construct

<400>  104
gggggcttta gtgctttagt gctt                                              24


<210>  105
<211>  24
<212>  DNA
<213>  Artificial

<220>
<223>  Synthetic Construct

<400>  105
gggggctaat gtgctaatgt gctt                                              24


<210>  106
<211>  24
<212>  DNA
<213>  Artificial

<220>
<223>  Synthetic Construct

<400>  106
tcgtcgaaaa gtcgaaaagt cgaa                                              24


<210>  107
<211>  24
<212>  DNA
<213>  Artificial

<220>
<223> Synthetic Construct

<400> 107
gggggtcttt ttttcttttt tttt 24

<210> 108
<211> 24
<212> DNA
<213> Artificial

<220>
<223> Synthetic Construct

<400> 108
tttttctttt ttttcttttt tggg 24

<210> 109
<211> 24
<212> DNA
<213> Artificial

<220>
<223> Synthetic Construct

<400> 109
aaaaatcttt ttttcttttt tttt 24

<210> 110
<211> 24
<212> DNA
<213> Artificial

<220>
<223> Synthetic Construct

<400> 110
tgctgctttt atgcttttat gctt 24

<210> 111
<211> 24
<212> DNA
<213> Artificial

<220>
<223> Synthetic Construct

<400> 111
tcatcattct gtcattctgt catt 24

<210> 112
<211> 24
<212> DNA
<213> Artificial

<220>
<223> Synthetic Construct

<400> 112
tttttctttt ttttcttttt tttt 24

<210>  113
<211>  24
<212>  DNA
<213>  Artificial

<220>
<223>  Synthetic Construct

<400>  113
tgctgctttt ctgcttttct gctt                                              24


<210>  114
<211>  24
<212>  DNA
<213>  Artificial

<220>
<223>  Synthetic Construct

<400>  114
tttttctttt tctttttttt tttt                                              24


<210>  115
<211>  24
<212>  DNA
<213>  Artificial

<220>
<223>  Synthetic Construct

<400>  115
   tttttccttt tttccttttt tttt                                              24


<210>  116
<211>  24
<212>  DNA
<213>  Artificial

<220>
<223>  Synthetic Construct

<400>  116
ccccctcttt ttttcttttt tttt                                              24


<210>  117
<211>  24
<212>  DNA
<213>  Artificial

<220>
<223>  Synthetic Construct

<400>  117
tttttctttt ttcttttttt tttt                                              24


<210>  118
<211>  24
<212>  DNA
<213>  Artificial

<220>
<223>  Synthetic Construct

<400>  118
   tttttctttt ttttcttttt tccc                                                24

<210>  119
<211>  24
<212>  DNA
<213>  Artificial

<220>
<223>  Synthetic Construct

<400>  119
   tttttctttt ttctcttttt ctct                                                24

<210>  120
<211>  24
<212>  DNA
<213>  Artificial

<220>
<223>  Synthetic Construct

<400>  120
tttttgcttt tttgcttttt tttt                                               24

<210>  121
<211>  24
<212>  DNA
<213>  Artificial

<220>
<223>  Synthetic Construct

<400>  121
   tttttacttt tttacttttt tttt                                              24

<210>  122
<211>  23
<212>  DNA
<213>  Artificial

<220>
<223>  Synthetic Construct

<400>  122
cataattttg taattttgtc att                                                23

<210>  123
<211>  24
<212>  DNA
<213>  Artificial

<220>
<223>  Synthetic Construct

<400>  123
   tttttctttt ttttcttttt taaa                                                24

<210>  124
<211>  24
<212>  DNA
<213>  Artificial

<220>
<223>  Synthetic Construct

<400>  124
   tttttctttt tttctttttt tttt                                                24


<210>  125
<211>  24
<212>  DNA
<213>  Artificial

<220>
<223>  Synthetic Construct

<400>  125
tttttttttt ttcattttgt gggg                                              24


<210>  126
<211>  24
<212>  DNA
<213>  Artificial

<220>
<223>  Synthetic Construct

<400>  126
tttttttttt ttcattttgt tttg                                              24


<210>  127
<211>  24
<212>  DNA
<213>  Artificial

<220>
<223>  Synthetic Construct

<400>  127
gggttttttt ttcattttgt tttt                                              24


<210>  128
<211>  24
<212>  DNA
<213>  Artificial

<220>
<223>  Synthetic Construct

<400>  128
gttttttttt ttcattttgt tttg                                              24


<210>  129
<211>  24
<212>  DNA
<213>  Artificial

<220>
<223>  Synthetic Construct

<400>  129
tttttttttt ttcattttgt ttgg                                              24

<210>  130
<211>  24
<212>  DNA
<213>  Artificial

<220>
<223>  Synthetic Construct

<400>  130
tttttttttt ttcattttgt ttta                                              24


<210>  131
<211>  24
<212>  DNA
<213>  Artificial

<220>
<223>  Synthetic Construct

<400>  131
tttttttttt ttcattttgt ttga                                              24


<210>  132
<211>  24
<212>  DNA
<213>  Artificial

<220>
<223>  Synthetic Construct

<400>  132
tttttttttt ttcattttgt ttag                                              24


<210>  133
<211>  24
<212>  DNA
<213>  Artificial

<220>
<223>  Synthetic Construct

<400>  133
tttttttttt ttcattttgt ttaa                                              24

SEQUENCE LISTING

<110>  IMMUNOTECH S.A.

<120>  Oligonucleotides stimulatory of the mesenchymal stem cell
       proliferation and uses thereof

<130>  05mg67e

<140>  EP05104854.4
<141>  2005-06-03

<160>  133

<170>  PatentIn version 3.3

<210>  1
<211>  24
<212>  DNA
<213>  Synthetic Construct

<400>  1
tgctgctttt gtgcttttgt gctt                                              24


<210>  2
<211>  24
<212>  DNA
<213>  Synthetic Construct

<400>  2
tcatcatttt gtcattttgt catt                                              24


<210>  3
<211>  24
<212>  DNA
<213>  Synthetic Construct

<400>  3
   tcctcctttt gtccttttgt cctt                                               24


<210>  4
<211>  24
<212>  DNA
<213>  Synthetic Construct

<400>  4
   tcttcttttt gtctttttgt cttt                                               24


<210>  5
<211>  24
<212>  DNA
<213>  Synthetic Construct

<400>  5
tagtagtttt gtagttttgt agtt                                              24


<210>  6
<211>  24
<212>  DNA
<213>  Synthetic Construct

<400>  6
tggtggtttt gtggttttgt ggtt                                              24

<210> 7
<211> 24
<212> DNA
<213> Synthetic Construct

<400> 7
ttgttgtttt gttgttttgt tgtt 24


<210> 8
<211> 24
<212> DNA
<213> Synthetic Construct

<400> 8
tgctgcaaaa gagcaaaaga gcaa 24


<210> 9
<211> 24
<212> DNA
<213> Synthetic Construct

<400> 9
tgctgccccc gcgcccccgc gccc 24


<210> 10
<211> 24
<212> DNA
<213> Synthetic Construct

<400> 10
tcattttttt gtttttttgt catt 24


<210> 11
<211> 24
<212> DNA
<213> Synthetic Construct

<400> 11
tcattgtttt gttgttttgt catt 24


<210> 12
<211> 24
<212> DNA
<213> Synthetic Construct

<400> 12
tcattctttt gttcttttgt catt 24


<210> 13
<211> 24
<212> DNA
<213> Synthetic Construct

<400> 13
aaaaaactaa aaaaaactaa aaaa 24


<210> 14
<211> 24
<212> DNA

<213>  Synthetic Construct

<400>  14
tcataatttt gtaattttgt catt                                              24


<210>  15
<211>  24
<212>  DNA
<213>  Synthetic Construct

<400>  15
tcattatttt gttattttgt catt                                              24


<210>  16
<211>  24
<212>  DNA
<213>  Synthetic Construct

<400>  16
tcatgatttt gtgattttgt catt                                              24


<210>  17
<211>  24
<212>  DNA
<213>  Synthetic Construct

<400>  17
tcatcctttt gtccttttgt catt                                              24


<210>  18
<211>  24
<212>  DNA
<213>  Synthetic Construct

<400>  18
   tcatcttttt gtctttttgt catt                                               24


<210>  19
<211>  24
<212>  DNA
<213>  Synthetic Construct

<400>  19
tttttttttt tttttttttt tttt                                              24


<210>  20
<211>  24
<212>  DNA
<213>  Synthetic Construct

<400>  20
cattttgttt tttttttttt tttt                                              24


<210>  21
<211>  24
<212>  DNA
<213>  Synthetic Construct

<400>  21
   ttcattttgt tttttttttt tttt                                               24

<210>  22
<211>  24
<212>  DNA
<213>  Synthetic Construct

<400>  22
ttttcatttt gttttttttt tttt                                              24


<210>  23
<211>  24
<212>  DNA
<213>  Synthetic Construct

<400>  23
   ttttttcatt ttgttttttt tttt                                                24


<210>  24
<211>  24
<212>  DNA
<213>  Synthetic Construct

<400>  24
ttttttttca ttttgttttt tttt                                              24


<210>  25
<211>  24
<212>  DNA
<213>  Synthetic Construct

<400>  25
tttttttttt cattttgttt tttt                                              24


<210>  26
<211>  24
<212>  DNA
<213>  Synthetic Construct

<400>  26
tttttttttt ttcattttgt tttt                                              24


<210>  27
<211>  24
<212>  DNA
<213>  Synthetic Construct

<400>  27
tttttttttt ttttcatttt gttt                                              24


<210>  28
<211>  24
<212>  DNA
<213>  Synthetic Construct

<400>  28
tttttttttt ttttttcatt ttgt                                              24


<210>  29
<211>  24
<212>  DNA
<213>  Synthetic Construct

<400>  29
ttttcatttt gtcattttgt tttt                                              24


<210>  30
<211>  24
<212>  DNA
<213>  Synthetic Construct

<400>  30
tcatcaattt gtcaatttgt catt                                              24


<210>  31
<211>  24
<212>  DNA
<213>  Synthetic Construct

<400>  31
tcatcatatt gtcatattgt catt                                              24


<210>  32
<211>  24
<212>  DNA
<213>  Synthetic Construct

<400>  32
tcatcattat gtcattatgt catt                                              24


<210>  33
<211>  24
<212>  DNA
<213>  Synthetic Construct

<400>  33
tcatcattta gtcatttagt catt                                              24


<210>  34
<211>  24
<212>  DNA
<213>  Synthetic Construct

<400>  34
tcatcatttt atcattttat catt                                              24


<210>  35
<211>  24
<212>  DNA
<213>  Synthetic Construct

<400>  35
tcatcatttt ttcatttttt catt                                              24


<210>  36
<211>  24
<212>  DNA
<213>  Synthetic Construct

<400>  36
tcatcatttt ctcattttct catt                                              24

<210>  37
<211>  24
<212>  DNA
<213>  Synthetic Construct

<400>  37
tcatcatttt gacattttga catt                                              24


<210>  38
<211>  24
<212>  DNA
<213>  Synthetic Construct

<400>  38
tcatcattta gacatttaga catt                                              24


<210>  39
<211>  24
<212>  DNA
<213>  Synthetic Construct

<400>  39
tcatcattat gacattatga catt                                              24


<210>  40
<211>  24
<212>  DNA
<213>  Synthetic Construct

<400>  40
tcatcatatt gacatattga catt                                              24


<210>  41
<211>  24
<212>  DNA
<213>  Synthetic Construct

<400>  41
tcatcattaa gacattaaga catt                                              24


<210>  42
<211>  24
<212>  DNA
<213>  Synthetic Construct

<400>  42
tcatcatata gacatataga catt                                              24


<210>  43
<211>  24
<212>  DNA
<213>  Synthetic Construct

<400>  43
tcatcataat gacataatga catt                                              24


<210>  44
<211>  24
<212>  DNA
<213>  Synthetic Construct

<400>  44
tcatcataaa gacataaaga catt                                              24


<210>  45
<211>  24
<212>  DNA
<213>  Synthetic Construct

<400>  45
tcatcaaaaa gacaaaaaga catt                                              24


<210>  46
<211>  24
<212>  DNA
<213>  Synthetic Construct

<400>  46
acatcatttt gtcattttgt catt                                              24


<210>  47
<211>  24
<212>  DNA
<213>  Synthetic Construct

<400>  47
ccatcatttt gtcattttgt catt                                              24


<210>  48
<211>  24
<212>  DNA
<213>  Synthetic Construct

<400>  48
gcatcatttt gtcattttgt catt                                              24


<210>  49
<211>  24
<212>  DNA
<213>  Synthetic Construct

<400>  49
taatcatttt gtcattttgt catt                                              24


<210>  50
<211>  24
<212>  DNA
<213>  Synthetic Construct

<400>  50
ttatcatttt gtcattttgt catt                                              24


<210>  51
<211>  24
<212>  DNA
<213>  Synthetic Construct

<400>  51
tgatcatttt gtcattttgt catt                                              24


<210>  52

<211>  24
<212>  DNA
<213>  Synthetic Construct

<400>  52
tcctcatttt gtcattttgt catt                                              24


<210>  53
<211>  24
<212>  DNA
<213>  Synthetic Construct

<400>  53
tcttcatttt gtcattttgt catt                                              24


<210>  54
<211>  24
<212>  DNA
<213>  Synthetic Construct

<400>  54
tcgtcatttt gtcattttgt catt                                              24


<210>  55
<211>  24
<212>  DNA
<213>  Synthetic Construct

<400>  55
tcaacatttt gtcattttgt catt                                              24


<210>  56
<211>  24
<212>  DNA
<213>  Synthetic Construct

<400>  56
tcaccatttt gtcattttgt catt                                              24


<210>  57
<211>  24
<212>  DNA
<213>  Synthetic Construct

<400>  57
tcagcatttt gtcattttgt catt                                              24


<210>  58
<211>  24
<212>  DNA
<213>  Synthetic Construct

<400>  58
tcatcatttt gtcattttgt aatt                                              24


<210>  59
<211>  24
<212>  DNA
<213>  Synthetic Construct

<400>  59

tcatcatttt gtcattttgt tatt 24

<210> 60
<211> 24
<212> DNA
<213> Synthetic Construct

<400> 60
tcatcatttt gtcattttgt gatt 24

<210> 61
<211> 24
<212> DNA
<213> Synthetic Construct

<400> 61
tcatcatttt gtcattttgt cctt 24

<210> 62
<211> 24
<212> DNA
<213> Synthetic Construct

<400> 62
tcatcatttt gtcattttgt cttt 24

<210> 63
<211> 24
<212> DNA
<213> Synthetic Construct

<400> 63
tcatcatttt gtcattttgt cgtt 24

<210> 64
<211> 24
<212> DNA
<213> Synthetic Construct

<400> 64
tcatcatttt gtcattttgt caat 24

<210> 65
<211> 24
<212> DNA
<213> Synthetic Construct

<400> 65
tcatcatttt gtcattttgt cact 24

<210> 66
<211> 24
<212> DNA
<213> Synthetic Construct

<400> 66
tcatcatttt gtcattttgt cagt 24

<210> 67
<211> 24

<212>  DNA
<213>  Synthetic Construct

<400>  67
tcatcatttt gtcattttgt cata                                              24


<210>  68
<211>  24
<212>  DNA
<213>  Synthetic Construct

<400>  68
tcatcatttt gtcattttgt catc                                              24


<210>  69
<211>  24
<212>  DNA
<213>  Synthetic Construct

<400>  69
tcatcatttt gtcattttgt catg                                              24


<210>  70
<211>  12
<212>  DNA
<213>  Synthetic Construct

<400>  70
ttttcatttt gt                                                           12


<210>  71
<211>  16
<212>  DNA
<213>  Synthetic Construct

<400>  71
ttttcatttt gttttt                                                       16


<210>  72
<211>  20
<212>  DNA
<213>  Synthetic Construct

<400>  72
ttttcatttt gttttttttt                                                   20


<210>  73
<211>  24
<212>  DNA
<213>  Synthetic Construct

<400>  73
tttttttttt ttcattttgt tttt                                              24


<210>  74
<211>  28
<212>  DNA
<213>  Synthetic Construct

<400>  74
ttttcatttt gttttttttt tttttttt                                          28

<210> 75
<211> 24
<212> DNA
<213> Synthetic Construct

<400> 75
tcatcaattg gtcaattggt catt 24


<210> 76
<211> 24
<212> DNA
<213> Synthetic Construct

<400> 76
tcatcaaatg gtcaaatggt catt 24


<210> 77
<211> 24
<212> DNA
<213> Synthetic Construct

<400> 77
tcatcaactg gtcaactggt catt 24


<210> 78
<211> 24
<212> DNA
<213> Synthetic Construct

<400> 78
tcatcaagtg gtcaagtggt catt 24


<210> 79
<211> 24
<212> DNA
<213> Synthetic Construct

<400> 79
tcatcaatag gtcaataggt catt 24


<210> 80
<211> 24
<212> DNA
<213> Synthetic Construct

<400> 80
tcatcaatcg gtcaatcggt catt 24


<210> 81
<211> 24
<212> DNA
<213> Synthetic Construct

<400> 81
tcatcaatgg gtcaatgggt catt 24


<210> 82
<211> 24
<212> DNA

<213>  Synthetic Construct

<400>  82
tcatcattat gtcattatgt catt                                              24


<210>  83
<211>  24
<212>  DNA
<213>  Synthetic Construct

<400>  83
tcatcattct gtcattctgt catt                                              24


<210>  84
<211>  24
<212>  DNA
<213>  Synthetic Construct

<400>  84
tcatcattgt gtcattgtgt catt                                              24


<210>  85
<211>  24
<212>  DNA
<213>  Synthetic Construct

<400>  85
tcatcattta gtcatttagt catt                                              24


<210>  86
<211>  24
<212>  DNA
<213>  Synthetic Construct

<400>  86
tcatcatttc gtcatttcgt catt                                              24


<210>  87
<211>  24
<212>  DNA
<213>  Synthetic Construct

<400>  87
tcatcatttg gtcatttggt catt                                              24


<210>  88
<211>  24
<212>  DNA
<213>  Synthetic Construct

<400>  88
tcatcatttt gacattttga catt                                              24


<210>  89
<211>  24
<212>  DNA
<213>  Synthetic Construct

<400>  89
tcatcatttt gccattttgc catt                                              24

<210> 90
<211> 24
<212> DNA
<213> Synthetic Construct

<400> 90
tcatcatttt ggcattttgg catt 24


<210> 91
<211> 24
<212> DNA
<213> Synthetic Construct

<400> 91
tgctgctttt gtgcttttgt gctt 24


<210> 92
<211> 24
<212> DNA
<213> Synthetic Construct

<400> 92
tcatcacttt ggcactttgg catt 24


<210> 93
<211> 24
<212> DNA
<213> Synthetic Construct

<400> 93
tcatcagttt ggcagtttgg catt 24


<210> 94
<211> 24
<212> DNA
<213> Synthetic Construct

<400> 94
tcatcatatt ggcatattgg catt 24


<210> 95
<211> 24
<212> DNA
<213> Synthetic Construct

<400> 95
tcatcatctt ggcatcttgg catt 24


<210> 96
<211> 24
<212> DNA
<213> Synthetic Construct

<400> 96
tcatcatgtt ggcatgttgg catt 24


<210> 97
<211> 24
<212> DNA
<213> Synthetic Construct

<400>  97
tgctgccccc gcgcccccgc gccc                                              24

<210>  98
<211>  24
<212>  DNA
<213>  Synthetic Construct

<400>  98
ggggtttttt tttttttttt tttt                                              24

<210>  99
<211>  24
<212>  DNA
<213>  Synthetic Construct

<400>  99
   ggggtctttt tttctttttt tctt                                              24

<210>  100
<211>  24
<212>  DNA
<213>  Synthetic Construct

<400>  100
tgctgccccc gcgcccccgc gccc                                              24

<210>  101
<211>  24
<212>  DNA
<213>  Synthetic Construct

<400>  101
ggggactttt atacttttat actt                                              24

<210>  102
<211>  24
<212>  DNA
<213>  Synthetic Construct

<400>  102
   ggggtatttt tttatttttt tatt                                              24

<210>  103
<211>  24
<212>  DNA
<213>  Synthetic Construct

<400>  103
gggggctttt gggcttttgg gcgg                                              24

<210>  104
<211>  24
<212>  DNA
<213>  Synthetic Construct

<400>  104
gggggcttta gtgctttagt gctt                                              24

<210>  105
<211>  24
<212>  DNA
<213>  Synthetic Construct

<400>  105
gggggctaat gtgctaatgt gctt                                              24


<210>  106
<211>  24
<212>  DNA
<213>  Synthetic Construct

<400>  106
tcgtcgaaaa gtcgaaaagt cgaa                                              24


<210>  107
<211>  24
<212>  DNA
<213>  Synthetic Construct

<400>  107
gggggtcttt ttttcttttt tttt                                              24


<210>  108
<211>  24
<212>  DNA
<213>  Synthetic Construct

<400>  108
tttttctttt ttttcttttt tggg                                              24


<210>  109
<211>  24
<212>  DNA
<213>  Synthetic Construct

<400>  109
aaaaatcttt ttttcttttt tttt                                              24


<210>  110
<211>  24
<212>  DNA
<213>  Synthetic Construct

<400>  110
tgctgctttt atgcttttat gctt                                              24


<210>  111
<211>  24
<212>  DNA
<213>  Synthetic Construct

<400>  111
tcatcattct gtcattctgt catt                                              24


<210>  112
<211>  24
<212>  DNA
<213>  Synthetic Construct

<400>  112
   tttttctttt ttttcttttt tttt                                                    24

<210>  113
<211>  24
<212>  DNA
<213>  Synthetic Construct

<400>  113
tgctgctttt ctgcttttct gctt                                                    24

<210>  114
<211>  24
<212>  DNA
<213>  Synthetic Construct

<400>  114
   tttttctttt tctttttttt tttt                                                    24

<210>  115
<211>  24
<212>  DNA
<213>  Synthetic Construct

<400>  115
   tttttccttt tttccttttt tttt                                                    24

<210>  116
<211>  24
<212>  DNA
<213>  Synthetic Construct

<400>  116
ccccctcttt ttttcttttt tttt                                                    24

<210>  117
<211>  24
<212>  DNA
<213>  Synthetic Construct

<400>  117
tttttctttt ttcttttttt tttt                                                    24

<210>  118
<211>  24
<212>  DNA
<213>  Synthetic Construct

<400>  118
tttttctttt ttttcttttt tccc                                                    24

<210>  119
<211>  24
<212>  DNA
<213>  Synthetic Construct

<400>  119
tttttctttt ttctcttttt ctct                                                    24

<210>  120

<211>  24
<212>  DNA
<213>  Synthetic Construct

<400>  120
tttttgcttt tttgcttttt tttt                                              24


<210>  121
<211>  24
<212>  DNA
<213>  Synthetic Construct

<400>  121
   tttttacttt tttacttttt tttt                                              24


<210>  122
<211>  23
<212>  DNA
<213>  Synthetic Construct

<400>  122
cataattttg taattttgtc att                                               23


<210>  123
<211>  24
<212>  DNA
<213>  Synthetic Construct

<400>  123
ttttttcttt ttttcttttt taaa                                              24


<210>  124
<211>  24
<212>  DNA
<213>  Synthetic Construct

<400>  124
   ttttttcttt tttctttttt tttt                                              24


<210>  125
<211>  24
<212>  DNA
<213>  Synthetic Construct

<400>  125
tttttttttt ttcattttgt gggg                                              24


<210>  126
<211>  24
<212>  DNA
<213>  Synthetic Construct

<400>  126
tttttttttt ttcattttgt tttg                                              24


<210>  127
<211>  24
<212>  DNA
<213>  Synthetic Construct

<400>  127
   gggttttttt ttcattttgt tttt                                                24

<210>  128
<211>  24
<212>  DNA
<213>  Synthetic Construct

<400>  128
gttttttttt ttcattttgt tttg                                                24

<210>  129
<211>  24
<212>  DNA
<213>  Synthetic Construct

<400>  129
tttttttttt ttcattttgt ttgg                                                24

<210>  130
<211>  24
<212>  DNA
<213>  Synthetic Construct

<400>  130
tttttttttt ttcattttgt ttta                                                24

<210>  131
<211>  24
<212>  DNA
<213>  Synthetic Construct

<400>  131
tttttttttt ttcattttgt ttga                                                24

<210>  132
<211>  24
<212>  DNA
<213>  Synthetic Construct

<400>  132
tttttttttt ttcattttgt ttag                                                24

<210>  133
<211>  24
<212>  DNA
<213>  Synthetic Construct

<400>  133
tttttttttt ttcattttgt ttaa                                                24

SEQUENCE LISTING

(1) GENERAL INFORMATION:

(i) APPLICANT: IMMUNOTECH S.A.

(ii) TITLE OF INVENTION: Oligonucleotides stimulatory of the mesenchymal stem cell

expansion and uses thereof

(iii) NUMBER OF SEQUENCES: 133

(iv) CORRESPONDENCE ADDRESS:

(A) ADDRESSEE: Dragotti & Associati

(B) STREET: Via Turati 32

(C) CITY: Milano

(D) STATE:

(E) COUNTRY: Italy

(F) ZIP: 20121

(v) COMPUTER READABLE FORM:

(A) MEDIUM TYPE: Floppy disk

(B) COMPUTER: IBM PC compatible

(vi) CURRENT APPLICATION DATA:

(A) APPLICATION NUMBER:

(B) FILING DATE:

(C) CLASSIFICATION:

(vii) ATTORNEY/AGENT INFORMATION:

(A) NAME: Roberto Pistolesi

(B) REGISTRATION NUMBER: 94880

(C) REFERENCE/DOCKET NUMBER: 05mg67e

(viii) TELECOMMUNICATION INFORMATION:

(A) TELEFONE: 0229014418

(B) TELEFAX: 0229003139

(2) INFORMATION FOR SEQ ID N°1 (IMT 021):

(i) SEQUENCE CHARACTERISTICS:

(A)LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 1:

TGCTGCTTTTGTGCTTTTGTGCTT

(3) INFORMATION FOR SEQ ID N°2 (IMT 504):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 2:

TCATCATTTTGTCATTTTGTCATT

(4) INFORMATION FOR SEQ ID N°3 (IMT 505):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 3:

TCCTCCTTTTGTCCTTTTGTCCTT

(5) INFORMATION FOR SEQ ID N°4 (IMT 506):

(i) SEQUENCE CHARACTERISTICS:

(A) LENGTH: 24 base pairs

(B) TYPE: nucleic acid

(C) STRANDEDNESS: single

(D) TOPOLOGY: linear

(ii)	MOLECULE TYPE: DNA
(iii)	SEQUENCE DESCRIPTION: SEQ ID Nº 4:
TCTTCTTTTTGTCTTTTTGTCTTT

(6) INFORMATION FOR SEQ ID Nº5 (IMT 501):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID Nº 5:
TAGTAGTTTTGTAGTTTTGTAGTT

(7) INFORMATION FOR SEQ ID Nº6 (IMT 502):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID Nº 6:
TGGTGGTTTTGTGGTTTTGTGGTT

(8) INFORMATION FOR SEQ ID Nº7 (IMT 503):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID Nº 7:
TTGTTGTTTTGTTGTTTTGTTGTT

(9) INFORMATION FOR SEQ ID N°8 (IMT 022):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 8:
TGCTGCAAAAGAGCAAAAGAGCAA

(10) INFORMATION FOR SEQ ID N°9 (IMT 023):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 9:
TGCTGCCCCCGCGCCCCCGCGCCC

(11) INFORMATION FOR SEQ ID N°10 (IMT 197):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 10:
TCATTTTTTTGTTTTTTTGTCATT

(12) INFORMATION FOR SEQ ID N°11 (IMT 198):
(i) SEQUENCE CHARACTERISTICS:

(A)LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 11:
TCATTGTTTTGTTGTTTTGTCATT

(13) INFORMATION FOR SEQ ID N°12 (IMT 199):
(i) SEQUENCE CHARACTERISTICS:
(A)LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 12:
TCATTCTTTTGTTCTTTTGTCATT

(14) INFORMATION FOR SEQ ID N°13 (IMT 509):
(i) SEQUENCE CHARACTERISTICS:
(A)LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 13:
AAAAAACTAAAAAAAACTAAAAAA

(15) INFORMATION FOR SEQ ID N°14 (IMT 531):
(i) SEQUENCE CHARACTERISTICS:
(A)LENGTH: 24 base pairs
(B) TYPE: nucleic acid
(C) STRANDEDNESS: single

(D) TOPOLOGY: linear
(ii) MOLECULE TYPE: DNA
(iii) SEQUENCE DESCRIPTION: SEQ ID N° 14:
TCATAATTTTGTAATTTTGTCATT
(16) INFORMATION FOR SEQ ID N°15 (IMT 532):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 15:
TCATTATTTTGTTATTTTGTCATT
(17) INFORMATION FOR SEQ ID N°16 (IMT 533):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 16:
TCATGATTTTGTGATTTTGTCATT
(18) INFORMATION FOR SEQ ID N°17 (IMT 534):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 17:

TCATCCTTTTGTCCTTTTGTCATT

(19) INFORMATION FOR SEQ ID N°18 (IMT 535):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 18:

TCATCTTTTTGTCTTTTTGTCATT

(20) INFORMATION FOR SEQ ID N°19 (IMT 053):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 19:

TTTTTTTTTTTTTTTTTTTTTTTT

(21) INFORMATION FOR SEQ ID N°20 (IMT 173):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 20:

CATTTTGTTTTTTTTTTTTTTTTT

(22) INFORMATION FOR SEQ ID N°21 (IMT 174):

(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 21:
TTCATTTTGTTTTTTTTTTTTTTT

(23) INFORMATION FOR SEQ ID N°22 (IMT 175):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 22:
TTTTCATTTTGTTTTTTTTTTTTT

(24) INFORMATION FOR SEQ ID N°23 (IMT 176):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 23:
TTTTTTCATTTTGTTTTTTTTTTT

(25) INFORMATION FOR SEQ ID N°24 (IMT 177):
(i)	SEQUENCE CHARACTERISTICS:
(A) LENGTH: 24 base pairs
(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 24:

TTTTTTTTCATTTTGTTTTTTTTT

(26) INFORMATION FOR SEQ ID N°25 (IMT 178):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 25:

TTTTTTTTTTCATTTTGTTTTTTT

(27) INFORMATION FOR SEQ ID N°26 (IMT 179):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 26:

TTTTTTTTTTTTCATTTTGTTTTT

(28) INFORMATION FOR SEQ ID N°27(IMT 180):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 27:

TTTTTTTTTTTTTTCATTTTGTTT

(29) INFORMATION FOR SEQ ID N°28 (IMT 181):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 28:

TTTTTTTTTTTTTTTTCATTTTGT

(30) INFORMATION FOR SEQ ID N°29 (IMT 182):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 29:

TTTTCATTTTGTCATTTTGTTTTT

(31) INFORMATION FOR SEQ ID N°30 (IMT 537):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 30:

TCATCAATTTGTCAATTTGTCATT

(32) INFORMATION FOR SEQ ID N°31 (IMT 538):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 31:
TCATCATATTGTCATATTGTCATT

(33) INFORMATION FOR SEQ ID N°32 (IMT 539):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 32:
TCATCATTATGTCATTATGTCATT

(34) INFORMATION FOR SEQ ID N°33 (IMT 540):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 33:
TCATCATTTAGTCATTTAGTCATT

(35) INFORMATION FOR SEQ ID N°34 (IMT 541):
(i) SEQUENCE CHARACTERISTICS:
(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 34:

TCATCATTTTATCATTTTATCATT

(36) INFORMATION FOR SEQ ID N°35 (IMT 542):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 35:

TCATCATTTTTTCATTTTTTCATT

(37) INFORMATION FOR SEQ ID N°36 (IMT 543):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 36:

TCATCATTTTCTCATTTTCTCATT

(38) INFORMATION FOR SEQ ID N°37 (IMT 544):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA
(iii) SEQUENCE DESCRIPTION: SEQ ID N° 37:
TCATCATTTTGACATTTTGACATT

(39) INFORMATION FOR SEQ ID N°38 (IMT 545):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 38:
TCATCATTTAGACATTTAGACATT

(40) INFORMATION FOR SEQ ID N°39 (IMT 546):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 39:
TCATCATTATGACATTATGACATT

(41) INFORMATION FOR SEQ ID N°40 (IMT 547):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 40:
TCATCATATTGACATATTGACATT

(42) INFORMATION FOR SEQ ID N°41 (IMT 548):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 41:
TCATCATTAAGACATTAAGACATT

(43) INFORMATION FOR SEQ ID N°42 (IMT 549):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 42:
TCATCATATAGACATATAGACATT

(44) INFORMATION FOR SEQ ID N°43 (IMT 550):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 43:
TCATCATAATGACATAATGACATT

(45) INFORMATION FOR SEQ ID N°44 (IMT 551):

(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 44:
TCATCATAAAGACATAAAGACATT

(46) INFORMATION FOR SEQ ID N°45 (IMT 552):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 45:
TCATCAAAAAGACAAAAAGACATT

(47) INFORMATION FOR SEQ ID N°46 (IMT 559):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 46:
ACATCATTTTGTCATTTTGTCATT

(48) INFORMATION FOR SEQ ID N°47 (IMT 560):
(A) SEQUENCE CHARACTERISTICS:
(A) LENGTH: 24 base pairs

(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 47:
CCATCATTTTGTCATTTTGTCATT

(49) INFORMATION FOR SEQ ID N°48 (IMT 561):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 48:
GCATCATTTTGTCATTTTGTCATT

(50) INFORMATION FOR SEQ ID N°49 (IMT 562):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 49:
TAATCATTTTGTCATTTTGTCATT

(51) INFORMATION FOR SEQ ID N°50 (IMT 563):
I. SEQUENCE CHARACTERISTICS:
(A) LENGTH: 24 base pairs
(B) TYPE: nucleic acid
(C) STRANDEDNESS: single

(D) TOPOLOGY: linear
II. MOLECULE TYPE: DNA
III. SEQUENCE DESCRIPTION: SEQ ID N° 50:
TTATCATTTTGTCATTTTGTCATT
(52) INFORMATION FOR SEQ ID N°51 (IMT 564):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 51:
TGATCATTTTGTCATTTTGTCATT
(53) INFORMATION FOR SEQ ID N°52 (IMT 565):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 52:
TCCTCATTTTGTCATTTTGTCATT
(54) INFORMATION FOR SEQ ID N°53 (IMT 566):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 53:

TCTTCATTTTGTCATTTTGTCATT

(55) INFORMATION FOR SEQ ID N°54 (IMT 567):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 54:

TCGTCATTTTGTCATTTTGTCATT

(56) INFORMATION FOR SEQ ID N°55 (IMT 568):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 55:

TCAACATTTTGTCATTTTGTCATT

(57) INFORMATION FOR SEQ ID N°56 (IMT 569):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 56:

TCACCATTTTGTCATTTTGTCATT

(58) INFORMATION FOR SEQ ID N°57 (IMT 570):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 57:
TCAGCATTTTGTCATTTTGTCATT

(59) INFORMATION FOR SEQ ID N°58 (IMT 571):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 58:
TCATCATTTTGTCATTTTGTAATT

(60) INFORMATION FOR SEQ ID N°59 (IMT 572):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 59:
TCATCATTTTGTCATTTTGTTATT

(61) INFORMATION FOR SEQ ID N°60 (IMT 573):
I. SEQUENCE CHARACTERISTICS:
(A) LENGTH: 24 base pairs
(B) TYPE: nucleic acid

(C) STRANDEDNESS: single
(D) TOPOLOGY: linear
II. MOLECULE TYPE: DNA
III. SEQUENCE DESCRIPTION: SEQ ID N° 60:
TCATCATTTTGTCATTTTGTGATT

(62) INFORMATION FOR SEQ ID N°61 (IMT 574):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 61:
TCATCATTTTGTCATTTTGTCCTT

(63) INFORMATION FOR SEQ ID N°62 (IMT 575):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 62:
TCATCATTTTGTCATTTTGTCTTT

(64) INFORMATION FOR SEQ ID N°63 (IMT 576):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA

III. SEQUENCE DESCRIPTION: SEQ ID N° 63:
TCATCATTTTGTCATTTTGTCGTT

(65) INFORMATION FOR SEQ ID N°64 (IMT 577):
I. SEQUENCE CHARACTERISTICS:
(A)LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 64:
TCATCATTTTGTCATTTTGTCAAT

(66) INFORMATION FOR SEQ ID N°65 (IMT 578):
I. SEQUENCE CHARACTERISTICS:
(A)LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 65:
TCATCATTTTGTCATTTTGTCACT

(67) INFORMATION FOR SEQ ID N°66 (IMT 579):
I. SEQUENCE CHARACTERISTICS:
(A)LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 66:
TCATCATTTTGTCATTTTGTCAGT

(68) INFORMATION FOR SEQ ID N°67 (IMT 580):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 67:
TCATCATTTTGTCATTTTGTCATA
(69) INFORMATION FOR SEQ ID N°68 (IMT 581):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 68:
TCATCATTTTGTCATTTTGTCATC
(70) INFORMATION FOR SEQ ID N°69 (IMT 582):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 69:
TCATCATTTTGTCATTTTGTCATG
(71) INFORMATION FOR SEQ ID N°70 (IMT 187):
I. SEQUENCE CHARACTERISTICS:
(A) LENGTH: 12 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 70:

TTTTCATTTTGT

(72) INFORMATION FOR SEQ ID N°71 (IMT 188):

I. SEQUENCE CHARACTERISTICS:

(A) LENGTH: 16 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 71:

TTTTCATTTTGTTTTT

(73) INFORMATION FOR SEQ ID N°72 (IMT 189):

I. SEQUENCE CHARACTERISTICS:

(A) LENGTH: 20 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 72:

TTTTCATTTTGTTTTTTTTT

(74) INFORMATION FOR SEQ ID N°73 (IMT 179):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 73:

TTTTTTTTTTTTCATTTTGTTTTT

(75) INFORMATION FOR SEQ ID N°74 (IMT 191):

I. SEQUENCE CHARACTERISTICS:

(A) LENGTH: 28 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 74:

TTTTCATTTTGTTTTTTTTTTTTTTTTT

(76) INFORMATION FOR SEQ ID N°75 (IMT 608):

I. SEQUENCE CHARACTERISTICS:

(E) LENGTH: 24 base pairs

(F) TYPE: nucleic acid

(G) STRANDEDNESS: single

(H) TOPOLOGY: linear

II. MOLECULE TYPE: DNA

(A) SEQUENCE DESCRIPTION: SEQ ID N° 75:

TCATCAATTGGTCAATTGGTCATT

(77) INFORMATION FOR SEQ ID N°76 (IMT 609):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 76:

TCATCAAATGGTCAAATGGTCATT

(78) INFORMATION FOR SEQ ID N°77 (IMT 610):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 77:
TCATCAACTGGTCAACTGGTCATT

(79) INFORMATION FOR SEQ ID N°78 (IMT 611):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 78:
TCATCAAGTGGTCAAGTGGTCATT

(80) INFORMATION FOR SEQ ID N°79 (IMT 612):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 79:
TCATCAATAGGTCAATAGGTCATT

(81) INFORMATION FOR SEQ ID N°80 (IMT 613):
I. SEQUENCE CHARACTERISTICS:

(I) LENGTH: 24 base pairs
(A) TYPE: nucleic acid
(B) STRANDEDNESS: single
(C) TOPOLOGY: linear
II. MOLECULE TYPE: DNA
III. SEQUENCE DESCRIPTION: SEQ ID N° 80:
TCATCAATCGGTCAATCGGTCATT
(82) INFORMATION FOR SEQ ID N°81 (IMT 614):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 81:
TCATCAATGGGTCAATGGGTCATT
(83) INFORMATION FOR SEQ ID N°82 (IMT 539):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 82:
TCATCATTATGTCATTATGTCATT
(84) INFORMATION FOR SEQ ID N°83 (IMT 597):
I. SEQUENCE CHARACTERISTICS:
(A) LENGTH: 24 base pairs
(B) TYPE: nucleic acid
(C) STRANDEDNESS: single

(D) TOPOLOGY: linear
II. MOLECULE TYPE: DNA
III. SEQUENCE DESCRIPTION: SEQ ID N° 83:
TCATCATTCTGTCATTCTGTCATT
(85) INFORMATION FOR SEQ ID N°84 (IMT 602):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 84:
TCATCATTGTGTCATTGTGTCATT
(86) INFORMATION FOR SEQ ID N°85 (IMT 540):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 85:
TCATCATTTAGTCATTTAGTCATT
(87) INFORMATION FOR SEQ ID N°86 (IMT 598):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 86:

TCATCATTTCGTCATTTCGTCATT

(88) INFORMATION FOR SEQ ID N°87 (IMT 603):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 87:

TCATCATTTGGTCATTTGGTCATT

(89) INFORMATION FOR SEQ ID N°88 (IMT 544):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 88:

TCATCATTTTGACATTTTGACATT

(90) INFORMATION FOR SEQ ID N°89 (IMT 599):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 89:

TCATCATTTTGCCATTTTGCCATT

(91) INFORMATION FOR SEQ ID N°90 (IMT 604):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 90:
TCATCATTTTGGCATTTTGGCATT

(92) INFORMATION FOR SEQ ID N°91 ( IMT21 )
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 91:
TGCTGCTTTTGTGCTTTTGTGCTT

(93) INFORMATION FOR SEQ ID N°92 (IMT 595):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 92:
TCATCACTTTGGCACTTTGGCATT

(94) INFORMATION FOR SEQ ID N°93 (IMT 600):
I. SEQUENCE CHARACTERISTICS:
(A) LENGTH: 24 base pairs

(B) TYPE: nucleic acid
(C) STRANDEDNESS: single
(D) TOPOLOGY: linear
II. MOLECULE TYPE: DNA
III. SEQUENCE DESCRIPTION: SEQ ID N° 93:
TCATCAGTTTGGCAGTTTGGCATT

(95) INFORMATION FOR SEQ ID N°94 (IMT 538):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 94:
TCATCATATTGGCATATTGGCATT

(96) INFORMATION FOR SEQ ID N°95 (IMT 596):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 95:
TCATCATCTTGGCATCTTGGCATT

(97) INFORMATION FOR SEQ ID N°96 (IMT 601):
I. SEQUENCE CHARACTERISTICS:
(A) LENGTH: 24 base pairs
(B) TYPE: nucleic acid
(C) STRANDEDNESS: single

(D) TOPOLOGY: linear

II. MOLECULE TYPE: DNA

III. SEQUENCE DESCRIPTION: SEQ ID N° 96:

TCATCATGTTGGCATGTTGGCATT

(98) INFORMATION FOR SEQ ID N°97 (IMT 023):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 97:

TGCTGCCCCCGCGCCCCCGCGCCC

(99) INFORMATION FOR SEQ ID N°98 (IMT 059):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 98

GGGGTTTTTTTTTTTTTTTTTTTT

(100) INFORMATION FOR SEQ ID N°99 (IMT 058):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 99:

GGGGTCTTTTTTTCTTTTTTTCTT

(101) INFORMATION FOR SEQ ID N°100 (IMT 037):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 100:

TGCTGCCCCCGCGCCCCCGCGCCC

(102) INFORMATION FOR SEQ ID N°101 (IMT 034):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 101:

GGGGACTTTTATACTTTTATACTT

(103) INFORMATION FOR SEQ ID N°102 (IMT 057):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 102:

GGGGTATTTTTTTATTTTTTTATT

(104) INFORMATION FOR SEQ ID N°103 (IMT 028):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 103:
GGGGGCTTTTGGGCTTTTGGGCGG
(105) INFORMATION FOR SEQ ID N°104 (IMT 040):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 104:
GGGGGCTTTAGTGCTTTAGTGCTT
(106) INFORMATION FOR SEQ ID N°105 (IMT 046):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 105:
GGGGGCTAATGTGCTAATGTGCTT
(107) INFORMATION FOR SEQ ID N°106 (IMT 518):
I. SEQUENCE CHARACTERISTICS:
(A) LENGTH: 24 base pairs
(B) TYPE: nucleic acid

(C) STRANDEDNESS: single

(D) TOPOLOGY: linear

II. MOLECULE TYPE: DNA

III. SEQUENCE DESCRIPTION: SEQ ID N° 106:

TCGTCGAAAAGTCGAAAAGTCGAA

(108) INFORMATION FOR SEQ ID N°107 ( IMT105)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°107:

GGGGGTCTTTTTTTCTTTTTTTTT

(109) INFORMATION FOR SEQ ID N°108 ( IMT104)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°108:

TTTTTTCTTTTTTTCTTTTTTGGG

(110) INFORMATION FOR SEQ ID N°109 ( IMT106)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°109:

AAAAATCTTTTTTTCTTTTTTTTT

(111) INFORMATION FOR SEQ ID N°110 ( IMT166)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°110:

TGCTGCTTTTATGCTTTTATGCTT

(112) INFORMATION FOR SEQ ID N°111 ( IMT597)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°111:

TCATCATTCTGTCATTCTGTCATT

(113) INFORMATION FOR SEQ ID N°112 ( IMT101)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°112:

TTTTTTCTTTTTTTCTTTTTTTTT

(114) INFORMATION FOR SEQ ID N°113 ( IMT169)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°113:

TGCTGCTTTTCTGCTTTTCTGCTT

(115) INFORMATION FOR SEQ ID N°114 ( IMT114)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°114:

TTTTTTCTTTTCTTTTTTTTTTTT

(116) INFORMATION FOR SEQ ID N°115 ( IMT111)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°115:

TTTTTCCTTTTTTCCTTTTTTTTT

(117) INFORMATION FOR SEQ ID N°116 ( IMT107)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°116:

CCCCCTCTTTTTTTCTTTTTTTTT

(118) INFORMATION FOR SEQ ID N°117 ( IMT113)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°117:

TTTTTTCTTTTTCTTTTTTTTTTT

(119) INFORMATION FOR SEQ ID N°118 ( IMT103)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°118:

TTTTTTCTTTTTTTCTTTTTTCCC

(120) INFORMATION FOR SEQ ID N°119 ( IMT186)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°119:
TTTTTTCTTTTTCTCTTTTTCTCT

(121) INFORMATION FOR SEQ ID N°120 ( IMT110)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°120:
TTTTTGCTTTTTTGCTTTTTTTTT

(122) INFORMATION FOR SEQ ID N°121 ( IMT109)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°121:
TTTTTACTTTTTTACTTTTTTTTT

(123) INFORMATION FOR SEQ ID N°122 ( IMT531)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°122:
CATAATTTTGTAATTTTGTCATT

(124) INFORMATION FOR SEQ ID N°123 ( IMT102)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°123:
TTTTTTCTTTTTTTCTTTTTTAAA

(125) INFORMATION FOR SEQ ID N°124 ( IMT112)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°124:
TTTTTTCTTTTTTCTTTTTTTTTT

(126) INFORMATION FOR SEQ ID N°125 (IMT 703)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°125:
TTTTTTTTTTTTCATTTTGTGGGG

(127) INFORMATION FOR SEQ ID N°126 (IMT 700)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°126:
TTTTTTTTTTTTCATTTTGTTTTG

(128) INFORMATION FOR SEQ ID N°127 (IMT 706)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°127:
GGGTTTTTTTTTCATTTTGTTTTT

(129) INFORMATION FOR SEQ ID N°128 (IMT 712)

I. SEQUENCE CHARACTERISTICS:

(E) LENGTH: 24 base pairs

(F) TYPE: nucleic acid

(G) STRANDEDNESS: single

(H) TOPOLOGY: linear

II. MOLECULE TYPE: DNA

III. SEQUENCE DESCRIPTION: SEQ ID N°128:

GTTTTTTTTTTTCATTTTGTTTTG

(130) INFORMATION FOR SEQ ID N°129 (IMT 701)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°126:

TTTTTTTTTTTTCATTTTGTTTGG

(131) INFORMATION FOR SEQ ID N°130 (IMT 713)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°130:

TTTTTTTTTTTTCATTTTGTTTTA

(132) INFORMATION FOR SEQ ID N°131 (IMT 719)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°131:

TTTTTTTTTTTTCATTTTGTTTGA

(133) INFORMATION FOR SEQ ID N°132 (IMT 720)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°132:

TTTTTTTTTTTTCATTTTGTTTAG

(134) INFORMATION FOR SEQ ID N°133 (IMT 710)

I. SEQUENCE CHARACTERISTICS:

(A)LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°133:

TTTTTTTTTTTTCATTTTGTTTAA

## Claims

1. Use of an oligonucleotide having about 14 to 100 nucleotides for the manufacture of a medicament for stimulating the proliferation of pluripotent mesenchymal stem cells.

2. Use according to claim1 **characterized in that** said oligonucleotide consists of 14 to 40 nucleotides.

3. Use according to claims 1 or 2 for the manufacture of a medicament for the treatment of tissue dysfunctions and/or for inducing tissue regeneration.

4. Use according to claims 1 or 2 for the manufacture of a medicament for the treatment of the tissue damage own to a heart stroke, for the treatment of skeletal deficiency or alteration, for the treatment of the tissue damage own to a cartilage deficiency or alteration, for the treatment of the tissue damage own to a nervous system deficiency or alteration, for the treatment of the tissue damage own to a adipose tissue deficiency or alteration, for the treatment of the tissue damage own to a liver deficiency or alteration, for the treatment of the tissue damage own to a muscle deficiency or alteration, for the treatment of the tissue damage own to a skin deficiency or alteration, for the treatment of the tissue damage own to a burn accident, for the treatment of the tissue damage own to a pancreas deficiency or alteration, for the treatment of the tissue damage own to a mucosal deficiency or alteration, for the treatment of the tissue damage own to a blood vessel deficiency or alteration, for the treatment of the tissue damage own to a blood deficiency or alteration and/or for improvement of bone marrow engraftment.

5. Use according to anyone of the above claims **characterized in that** said oligonucleotide consists of 14 to 26 nucleotides.

6. Use according to anyone of the above claims **characterized in that** said oligonucleotide has any kind of modification of the natural (phosphodiester) phosphate backbone.

7. Use according to anyone of the above claims **characterized in that** said oligonucleotide has a phosphate backbone modification on the 5' internucleotide linkages.

8. Use according to anyone of the above claims **characterized in that** said oligonucleotide has a phosphate backbone modification on the 3' internucleotide linkages.

9. Use according to anyone of the above claims **characterized in that** said oligonucleotide has at least one of the internucleotide linkages as a phosphorotioate linkage.

10. Use according to anyone of the above claims **characterized in that** said oligonucleotide has at least one subsequence with the following composition: PyNTTTTNT, wherein Py is C or T and wherein N is any deoxyribonucleotide.

11. Use according to anyone of the above claims **characterized in that** said oligonucleotide is selected from the group consisting of:
TCATCATTTTGTCATTTTGTCATT (SEQ ID N°2);
TCCTCCTTTTGTCCTTTTGTCCTT (SEQ ID N°3);
TCTTCTTTTTGTCTTTTTGTCTTT (SEQ ID N°4);
TTGTTGTTTTGTTGTTTTGTTGTT (SEQ ID N°7);
TCATTTTTTTGTTTTTTTGTCATT (SEQ ID N°10);
TCATTGTTTTGTTGTTTTGTCATT (SEQ ID N°11);
TCATTCTTTTGTTCTTTTGTCATT (SEQ ID N°12);
TCATTATTTTGTTATTTTGTCATT (SEQ ID N°15);
TCATCCTTTTGTCCTTTTGTCATT (SEQ ID N°17);
TCATCTTTTTGTCTTTTTGTCATT (SEQ ID N°18);
CATTTTGTTTTTTTTTTTTTTTTT (SEQ ID N°20);
TTCATTTTGTTTTTTTTTTTTTTT (SEQ ID N°21);
TTTTCATTTTGTTTTTTTTTTTTT (SEQ ID N°22);
TTTTTTCATTTTGTTTTTTTTTTT (SEQ ID N°23);
TTTTTTTTCATTTTGTTTTTTTTT (SEQ ID N°24);
TTTTTTTTTTCATTTTGTTTTTTT (SEQ ID N°25);
TTTTTTTTTTTTCATTTTGTTTTT (SEQ ID N°26);
TTTTTTTTTTTTTTCATTTTGTTT (SEQ ID N°27);
TTTTTTTTTTTTTTTTCATTTTGT (SEQ ID N° 28);
TTTTCATTTTGTCATTTTGTTTTT (SEQ ID N°29);
TCATCAATTTGTCAATTTGTCATT (SEQ ID N°30);
ACATCATTTTGTCATTTTGTCATT (SEQ ID N°46);
CCATCATTTTGTCATTTTGTCATT (SEQ ID N°47);
GCATCATTTTGTCATTTTGTCATT (SEQ ID N°48);
TAATCATTTTGTCATTTTGTCATT (SEQ ID N°49);
TTATCATTTTGTCATTTTGTCATT (SEQ ID N°50);
TGATCATTTTGTCATTTTGTCATT (SEQ ID N°51);
TCCTCATTTTGTCATTTTGTCATT (SEQ ID N°52);
TCTTCATTTTGTCATTTTGTCATT (SEQ ID N°53);
TCAACATTTTGTCATTTTGTCATT (SEQ ID N°55);
TCACCATTTTGTCATTTTGTCATT (SEQ ID N°56);
TCAGCATTTTGTCATTTTGTCATT (SEQ ID N°57);
TCATCATTTTGTCATTTTGTAATT (SEQ ID N°58);
TCATCATTTTGTCATTTTGTTATT (SEQ ID N°59);
TCATCATTTTGTCATTTTGTGATT (SEQ ID N°60);
TCATCATTTTGTCATTTTGTCCTT (SEQ ID N°61);
TCATCATTTTGTCATTTTGTCTTT (SEQ ID N°62);
TCATCATTTTGTCATTTTGTCAAT (SEQ ID N°64);
TCATCATTTTGTCATTTTGTCACT (SEQ ID N°65);
TCATCATTTTGTCATTTTGTCAGT (SEQ ID N°66);
TCATCATTTTGTCATTTTGTCATA (SEQ ID N°67);
TCATCATTTTGTCATTTTGTCATC (SEQ ID N°68);
TCATCATTTTGTCATTTTGTCATG (SEQ ID N°69);
TCATCAATTGGTCAATTGGTCATT (SEQ ID N°75);
TCATCAACTGGTCAACTGGTCATT (SEQ ID N°77);
TCATCATTGTGTCATTGTGTCATT (SEQ ID N°84);
TCATCATTTGGTCATTTGGTCATT (SEQ ID N°87);
TGCTGCTTTTGTGCTTTTGTGCTT (SEQ ID N°91);
TCATCATCTTGTCATCTTGTCATT (SEQ ID N°95);
TCATCATGTTGTCATGTTGTCATT (SEQ ID N°96);
GGGGGTCTTTTTTTCTTTTTTTTT (SEQ ID N°107);
TTTTTTCTTTTTTTCTTTTTTGGG (SEQ ID N°108);
AAAAATCTTTTTTTCTTTTTTTTT (SEQ ID N°109);
TGCTGCTTTTATGCTTTTATGCTT (SEQ ID N°110);
TCATCATTCTGTCATTCTGTCATT (SEQ ID N°111);
TTTTTTCTTTTTTTCTTTTTTTTT (SEQ ID N°112);
TGCTGCTTTTCTGCTTTTCTGCTT (SEQ ID N°113);
TTTTTTCTTTTCTTTTTTTTTTTT (SEQ ID N°114);
TTTTTCCTTTTTTCCTTTTTTTTT (SEQ ID N°115);
CCCCCTCTTTTTTTCTTTTTTTTT (SEQ ID N°116);
TTTTTTCTTTTTCTTTTTTTTTTT (SEQ ID N°117);
TTTTTTCTTTTTTTCTTTTTTCCC (SEQ ID N°118);
TTTTTTCTTTTTCTCTTTTTCTCT (SEQ ID N°119);
TTTTTGCTTTTTTGCTTTTTTTT (SEQ ID N°120);
TTTTTACTTTTTTACTTTTTTTTT (SEQ ID N°121);
TCATAATTTTGTAATTTTGTCATT (SEQ ID N°122);
TTTTTTCTTTTTTTCTTTTTTAAA (SEQ ID N°123);
TTTTTTCTTTTTTCTTTTTTTTTT (SEQ ID N°124);
TTTTTTTTTTTTCATTTTGTGGGG (SEQ ID N°125);
TTTTTTTTTTTTCATTTTGTTTTG (SEQ ID N°126);
GGGTTTTTTTTTCATTTTGTTTTT (SEQ ID N°127);
GTTTTTTTTTTTCATTTTGTTTTG (SEQ ID N°128);
TTTTTTTTTTTTCATTTTGTTTGG (SEQ ID N°129);
TTTTTTTTTTTTCATTTTGTTTTA (SEQ ID N°130).
TTTTTTTTTTTTCATTTTGTTTGA (SEQ ID N°131);
TTTTTTTTTTTTCATTTTGTTTAG (SEQ ID N°132);
TTTTTTTTTTTTCATTTTGTTTAA (SEQ ID N°133).

12. Use according to anyone of the above claims **characterized in that** said medicament is administered to a human.

13. Use according to anyone of the above claims **characterized in that** said oligonucleotide is included in a pharmaceutically acceptable carrier.

14. Use according to anyone of the above claims **characterized in that** said oligonucleotide is encapsulated in a slow release delivery vehicle.

15. Use according to anyone of the above claims **characterized in that** said oligonucleotide is administered to a subject combined with a device aimed to aid in the treatment of a tissue or organ deficiency or alteration.

16. Use according to anyone of the above claims **characterized in that** said oligonucleotide is present in amounts of 1 µg to 100mg per dose.

17. Use according to anyone of the above claims **characterized in that** said medicament is selected from the group consisting of liquid, gel and lyophilized formulations.

18. Use according to anyone of the above claims **characterized in that** said medicament is administered by intradermic, intramuscular or intravenous injection.

19. Use according to anyone of the above claims **characterized in that** said medicament is administered by an oral, intranasal, anal, vaginal, or trans-dermal route.

20. Use according to anyone of the above claims **characterized in that** the mesenchymal stem cells are obtained from bone marrow.

21. Use according to anyone of the above claims **characterized in that** the mesenchymal stem cells are obtained from periosteum.

22. Use according to anyone of the above claims **characterized in that** the mesenchymal stem cells differentiate into cells of more than one tissue cell type.

23. Use according to claim 22 **characterized in that** said tissue is selected from bone, cartilage, adipose, tendon, ligament, dermis, epidermis, neuronal, cardiac, renal, glandular and hepatic tissue.
